(19) Europäisches Patentamt
European Patent Office
Office européen des brevets

(11) Veröffentlichungsnummer: **0 033 503**
**B1**

(12) **EUROPÄISCHE PATENTSCHRIFT**

(45) Veröffentlichungstag der Patentschrift: **10.09.86**

(21) Anmeldenummer: **81100544.6**

(22) Anmeldetag: **26.01.81**

(51) Int. Cl.⁴: **C 07 D 405/06,**
C 07 D 487/04,
C 07 D 403/06, A 61 K 31/335

# (54) Arzneimittel mit cytostatischer Wirkung sowie Verwendung von mehrfach mit Glycidylgruppen substituierten N-heterocyclischen Ringverbindungen in pharmazeutischen Zubereitungen.

(30) Priorität: **31.01.80 DE 3003357**
**31.01.80 DE 3003356**
**31.01.80 DE 3003404**
**10.03.80 AT 1331/80**
**10.03.80 AT 1330/80**
**12.03.80 AT 1365/80**
**27.03.80 AT 1649/80**
**17.11.80 AT 5644/80**

(43) Veröffentlichungstag der Anmeldung:
**12.08.81 Patentblatt 81/32**

(45) Bekanntmachung des Hinweises auf die Patenterteilung:
**10.09.86 Patentblatt 86/37**

(84) Benannte Vertragsstaaten:
**AT BE CH DE FR GB IT LI NL SE**

(56) Entgegenhaltungen:
**EP-A-0 014 981**
**FR-M- 7 153**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Fischer, Herbert, Dr.**
**Kamper Weg 139**
**D-4000 Düsseldorf 12 (DE)**
Erfinder: **Möller, Hinrich, Dr.**
**Schumannstrasse 11**
**D-4019 Monheim (DE)**
Erfinder: **Budnowski, Manfred, Dr.**
**Am Nettchesfeld 24**
**D-4000 Düsseldorf 13 (DE)**
Erfinder: **Hase, geb. Kornrumpf, Brigitte, Dr.**
**Millrather Weg 29**
**D-4006 Erkrath 1 (DE)**
Erfinder: **Wilk, Hans-Christoph, Dr.**
**Görlitzer Strasse 5**
**D-4040 Neuss (DE)**
Erfinder: **Zeidler, Ulrich, Dr.**
**Heinrich-Lersch-Strasse 19**
**D-4000 Düsseldorf (DE)**
Erfinder: **Hase, Christian, Dr.**
**Millrather Weg 29**
**D-4006 Erkrath 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 033 503 B1

**Beschreibung**

Es ist bekannt, daß eine Reihe von alkylierenden Substanzen eine cytostatische beziehungsweise cytotoxische Wirkung entfalten. Die bekanntesten Verbindungen leiten sich vom sogenannten Stickstofflost ab. Darüber hinaus ist es auch bekannt, wenigstens zwei Epoxidgruppen im Molekül enthaltende Verbindungen als Cancerostatika zu verwenden. Derartige Verbindungen sind beispielsweise das 4,4'-Bis-(2,3-epoxypropyl)-di-piperidinyl-(1,1') und das 1,2-15,16-Diepoxy-4,7-10,13-tetraoxohexadecan. Allerdings haben die letzteren Verbindungen keine wesentliche Verbesserung in der cytostatischen Behandlung gebracht und werden kaum verwendet.

Gegenstand der nicht vorveröffentlichten EP—A—0 014 981 sind Arzneimittelzubereitungen mit cytostatischer Wirksamkeit, die als Wirkstoff Triglycidylisocyanurat (TGI) und/oder solche TGI-Derivate enthalten, in denen das Wasserstoffatom des Kohlenstoffs in 2-Stellung der Glycidylgruppe durch einen Alkylrest mit 1 bis 4 Kohlenstoffatomen ersetzt sein kann. Mit anderen Worten bedeutet dies, daß die drei N-Atome des Isocyanursäurerings mit Epoxygruppen enthaltenden Glycidylresten substituiert sind, die in 2-Stellung auch mit einem Alkylrest mit 1 bis 4 Kohlenstoffatomen substituiert sein können.

Gegenstand der älteren AT—PA—371 816 sind u.a. die Herstellung von Verbindungen der allgemeinen Formel

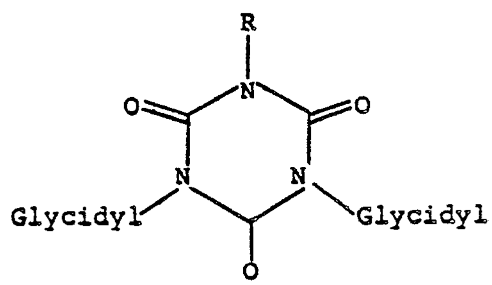

in der R die folgende Bedeutung hat: Alkyl, Aryl, Aralkyl, Alkaryl, Cycloalkyl, welche Reste gewünschtenfalls auch ungesättigt und/oder substituiert sein können, cytostatische Wirksamkeit aufweisen.

Aus der FR—A—7.153 M ist bekannt, daß die Verbindung 1,3-Bis-(glycidyl)-5,5-dimethylhydantoin im Tierversuch das Wachstum des Walker-Tumors hemmt. Doch kann der Fachmann daraus noch keinen Rückschluß auf die Wirksamkeit von heterocyclischen Verbindungen N-ständigen Glycidylgruppen ziehen, bei denen der Heterocyclus erfindungsgemäß abgewandelt worden ist. Insbesondere war nicht vorauszusagen, daß außer der häufig beobachteten Wachstumsinhibierung dieses Modelltumors mit der genannten Substanzklasse auch eine Lebensverlängerung bei Versuchstieren, denen der Test Tumor P 388 inplantiert worden ist, beobachtet werden kann.

Die vorliegende Erfindung geht von der Festellung aus, daß der N-Glycidylgruppierung ganz allgemein hohe cytostatische Wirksamkeit dann zukommt, wenn bestimmte und im folgenden geschilderte Bedingungen für den Einbau solcher N-Glycidylgruppen in das Molekül des Wirkstoffs erfüllt sind.

Gegenstand der Erfindung sind somit Arzneimittelzubereitungen mit cytostatischer Wirksamkeit auf Basis von Polyglycidyl-substituierten N-heterocyclischen organischen Ringverbindungen, dadurch gekennzeichnet, daß sie in einem heterocyclischen Ring oder in zwei miteinander verbundenen heterocyclischen Ringen, die entweder ein oder zwei Ringglieder gemeinsam haben oder durch ein Brückenglied miteinander verknüpft sind, wenigstens 2 Ringsegmente der Formel

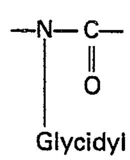

oder wenigstens ein Ringsegment der Formel

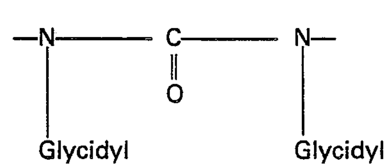

enthalten, in denen der Glycidylrest der Formel

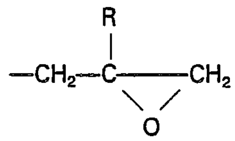

entspricht, in der R Wasserstoff oder der Methylrest ist, und daß sie weiterhin, sofern diese Ringsegmente nicht selbst zum Ring geschlossen sind, Kohlenstoff und gegebenenfalls zusätzlich Stickstoff als Ringschlußglieder enthalten, und daß sie als weitere Substituenten an den heterocyclischen Ringen Halogene oder unsubstituierte Kohlenwasserstoffreste mit nicht mehr als 8 C-Atomen aufweisen können, wobei als Heterocyclen Isocyanurate, Urazole und Hydantoine ausgenommen sind und wobei die darin vorliegenden Polyglycidyl-substituierten N-Heterocyclen das Molekulargewicht von 1 000 nicht überschreiten.

Es wurde überraschenderweise, gefunden, daß unter den vorstehend beschriebenen strukturellen Bedinungen ganz allgemein N-heterocyclischen organischen Ringverbindungen dann cytostatische Wirksamkeit zukommt, wenn zwei oder mehr N-Glycidylgruppierungen im Molekül enthalten sind, wobei zwingend diese N-Glycidylgruppen Teile einer Amid- und/oder Imidstruktur sind. Das die Glycidylgruppe tragende Stickstoffatom steht also in Nachbarstellung zu wenighstens einer Carbonylgruppe und dieses System aus Carbonylgruppe und mit einer Glycidylgruppe substituiertem Stickstoff und gegebenenfalls einer weiteren Carbonylgruppe ist in das heterocyclische Ringsystem eingebunden, ist also unmittelbar Ringbestandteil.

Wenn auch der Wirkungsmechanismus der im Rahmen der Erfindung eingesetzten Verbindungen im einzelnen nicht geklärt ist, so kann doch vermutet werden, daß die Bedeutung des restlichen Molekülbestandteiles insbesondere im Einfluß auf die Löslichkeit beziehungsweise auf die Verteilung der lipophilen und hydrophilen Präferenzen liegt. Hierdurch erklärt sich die breite Definition der erfindungsgemäß einsetzbaren Wirkstoffe mit cytostatischer Aktivität, die als übereinstimmendes Struktur- und Wirkungselement stets wenigstens zwei N-Glycidylamid- und/oder N-Glycidylimid-Funktionen aufweisen.

Die erfindungsgemäß zum Einsatz kommenden mehrfach mit Glycidylgruppen substituierten N-heterocyclischen organischen Ringverbundungen lassen sich genauer derart kennzeichnen, daß siewenigstens zwei Ringsegmente der Formel

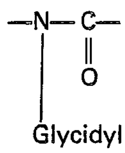

oder wenigstens ein Ringsegment der Formel I

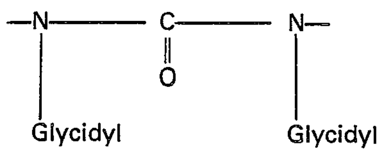

(I)

aufweisen, und daß sie weiterhin-sofern diese Ringsegmente nicht selber zum Ring geschlossen sind-Kohlenstoff und gegebenenfalls zusätzlichen Stickstoff, als Ringschlußglieder enthalten. Der Glycidylrest entspricht dabei der zuvor angegebenen allgemeinen Formel.

Die neben den formelmäßig dargestellten Ringsegmenten gegebenenfalls vorliegenden zusätzlichen Ringschlußglieder sind Kohlenstoff und gegebenenfalls zusätzlicher Stickstoff. Der Kohlenstoff kann dabei als Carbonylgruppe aber auch als Kohlenwasserstoffgruppe vorliegen, die — unter Berücksichtigung der im folgenden noch angegebenen bevorzugten Definitionen — auch substituiert sein kann.

Es hat sich weiterhin gezeigt, daß sur Schaffung ausgeprägter cytostatischer Wirksamkeiten zweckmäßigerweise bestimmte maximale Molekulargewichte der erfindungsgemäß eingesetzten Verbindungen nicht überschritten werden. Im allgemeinen liegt das Molekulargewicht der cytostatisch aktiven Komponente nicht über etwa 1000. Bevorzugt werden Verbindungen mit einem Molekulargewicht bis zu etwa 750. Meist als besonders wirksam haben sich solche mehrere Glycidylsubstituenten enthaltende N-Heterocyclen erwiesen, deren Molekulargewicht den Grenzwert von etwa 500 nicht oder nur mäßig überschreitet — z.B. den Wert von 550 oder höchstens 600 erreicht — und insbesondere solche im Bereich von etwa 200 bis 500, z.B. von etwa 300 bis 500. Diese Zahlenangaben gelten in erster Linie für N-heterocyclische Verbindungen der angegebenen Art mit 2 bis 4, insbesondere 2 oder 3 Glycidylgruppen in Amid- oder Imid-Bindung.

Im Rahmen der erfindungsgemäß eingesetzten Wirkstoffe mit cytostatischer Aktivatät lassen sich verschiedene Grundtypen unterschieden. In einer ersten Unterklasse liegen Heterocyclen mit nur einem heterocyclischen Ring im Molekül vor. Dieser Ring enthält dann die zuvor definierten N-Glycidylsubstituentern an Amid- beziehungsweise Imidstrukturen, gegebenenfalls neben weiteren substituierten oder unsubstituierten Ringgliedern der angegebenen Art. In diesem Fall liegen auf jeden Fall an diesem einen Ring wenigstens zwei Glycidylreste an Amid und/oder Imid-Stickstoffatomen vor.

In einer weiteren Unterklasse ist die erfindungsgemäß eingesetzte cytostatisch wirksame Ringverbindung ihrerseits aus mehreren miteinander verbundenen Ringen aufgebaut. Hierbei lassen sich zwei charakteristische Unterklassen unterschieden: In der ersten Unterklasse sind zwei individuelle heterocyclische Ringsysteme vorgesehen, die jedoch durch ein Brückenglied miteinander verbunden sind. In der zweiten Unterklasse sind zwei heterocyclische Ringsysteme derart miteinander verschmolzen, daß ein, zwei Ringglieder jeweils zwei solchen Ringen gemeinsam sind. In beiden hier geschilderten Unterklassen besteht die Möglichkeit, daß jeweils nur eine N-Glycidylgruppe der geschilderten Art pro heterocyclischem Ringsystem vorliegt, vorausgesetzt, daß das Molekül als Ganzes wenigstens zwei dieser cytostatisch aktiven Gruppen besitzat. Es ist allerdings möglich, daß einer oder mehrere der individuellen Ringe des insgesamt vorliegenden Mehrringsystems mehr als nur einen Glycidyl-N-Gruppierung in Amid- oder Imid-Bindung enthält. Im übrigen gilt bezüglich der restlichen Ringglieder und sonstigen Molekülbestandteile, daß ihre Beschaffenheit grundsätzlich von untergeordneter Bedeutung ist und vermutlich im wesentlichen Einfluß auf die Verteilung des Wirkstoffes im Organismus nimmt.

Die mit Glycicylgruppen substituierten N-heterocyclischen Ringe sind in den erfindungsgemäß eingesetzten Wirkstoffen gesättigt oder enthalten Doppelbindungen. Die Anzahl der Ringglieder ist wiederum von untergeordneter Bedeutung und wird vorwiegend durch die Zugänglichkeit und durch Stabilitätsüberlegungen bestimmt. Bevorzugt wiesen die einzelnen Ringe nicht mehr als 14 und insbesondere nicht mehr als 12 Ringglieder auf. Als untere Grenze kann aus Überlegungen zur Ringstabilität die Gliederzahl von 4 gelten. Besondere Bedeutung kommt Ringsystemen mit einer Ringgliederzahl bis zu 10 zu, wobei — in erster Linie aus Gründen der präparativen Zugänglichkeit — Ringverbindungen einer Ringgliederzahl von 5 bis 7 besondere Bedeutung haben. Heterocyclen mit 5 oder 6 Kohlenstoffatomen und dabei N-H-Gruppierungen in Amid- oder Imid-Bindung sind in großer Zahl bekannt. Sie sind meist der Glycidierung am N-Atom zugänglich — entweder über Umsetzung mit Epihalogenhydrin oder Einführung eines Allylrestes mit anschließender Epoxidierung der Doppelbindung. Verbindungen dieser Art sind dementsprechend von vorrangigem Interesse für die Erfindung.

Erfindungsgemäß, besonders geeignete Heterocyclen-Strukturen lassen sich damit einer Reihe von Unterklassen zuordnen. Eine erste Unterklasse sind die Cyclischen Ureide, siehe hierzu beispielsweise FIESER/FIESER "Lehrbuch der organischen Chemie" Verlag Chemie GmbH, Weinheim (1954) S. 254 bis 260. Die erfindungsgemäß geeigneten cyclischen Ureide enthalten in der Vorstufe vor der Einführung der Glycidylreste wenigstens 2 —NH-Gruppen in Nachbarstellung zu einer Oxogruppe-CO—. Durch Einführung von wenigstens 2 Glycidylresten in N-Substitution und in Nachbarstellung zur Oxogruppe entstehen erfindungsgemäß besonders bevorzugte Verbindungen mit cytostatischer Wirksamkeit.

Die cyclischen Ureide können Verbindungen mit einem oder mit mehreren heterocyclischen Ringsystem sein. Beim Vorliegen mehrerer heterocyclischer Ringsysteme können diese miteinander verschmolzen oder lediglich mittels eines Bindegliedes miteinander verbunden sein. Die bevorzugte Ringgliederzahl liegt bei 5 bis 7, insbesondere 5 und/oder 6.

Eine weitere besonders bevorzugte Unterklasse der erfindungsgemäßen Wirkstoffe lietet sich von cyclischen Imiden ab, wie sie im nachfolgenden (allgemeine Formel VII) ausführlich diskutiert werden.

Eine weitere Unterklasse der erfindungsgemäß geeigneten Verbindungen sind N-Glycidyl-substituierte cyclische Amide mit wenigstens 2 N-Glycidyl-substituierten Amidgruppen im Molekül. Schließlich sind Mischtypen der hier einzeln aufgeführten Untergruppen möglich.

In besonders wichtigen Ausführungsformen der Erfindung liegen damit in den Wirkstoffgemischen mit Glycidylgruppen substituierte N-heterocyclische Ringverbindungen der folgenden allgemeinen Formeln vor:

a)

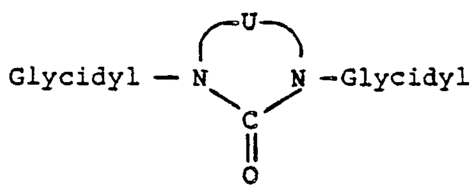

In dieser Formel ist U eine zweibindige Gruppe, die den Heterocyclus zu einem 5- bis 7-gliedrigen Ring, insbesondere einem 5- oder 6-gliedrigen Ring schließt und als Ringglieder C, N, O und/oder S aufweist.

b)

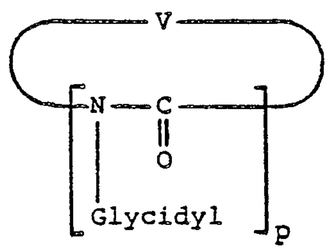

In dieser allgemeinen Formel entspricht die Bedeutung von V der zuvor zu U angegebenen Bedeutung aus dem Formelbild zu a), p ist eine Zahl von 2 oder 3, bevorzugt 2. Die beiden Glycidyl-substituierten Carbonamidgruppen können unmittelbar aneinander anschließen, sie können aber auch durch ein Zwischenglied voneinander getrennt sein.

c)

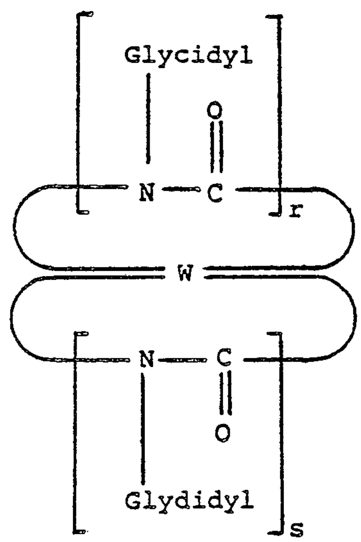

In dieser allgemeinen formel ist W eine 4-bindige Gruppe die die beiden Heterocyclen zu 5- bis 7-gliedrigen Ringen — insbesondere zu 5- und/oder 6-gliedrigen Ringen — schließt und als Ringglieder C, oder N aufweist. r und s bedeuten Zahlen von 1 bis 3, insbesondere 1 oder 2. r und s können dabei gleich oder verschieden sein. Besondere Bedeutung haben Verbindungen, in denen 4 und s jeweils 1 bedeuten.

d)

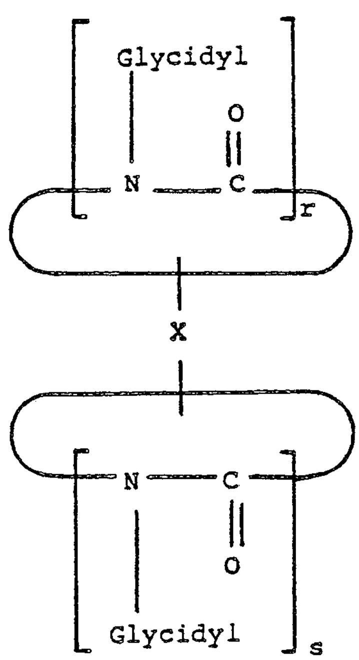

In dieser allgemeinen Formel ist X eine 2-bindige Gruppe, die 2 heterocyclische Ringsysteme der unter b) geschilderten Art miteinander verbindet. X weist dabei vorzugsweise nicht mehr als 8, insbesondere nicht mehr als 6 Glieder auf. r und s haben die zu c) erläuterte Bedeutung. In der hier dargestellten Verbindungesklasse kann das Bindeglied X auch mit einem oder mit 2 heterocyclischen Resten besetzt sein, die dem zuvor dargestellten Formelbild a) entsprechen.

Eventuelle Substituenten an den N-heterocyclischen Ringen — beziehungsweise an den in diesen Ringen vorliegenden zusätzlichen Ringschlußgliedern — oder an Ringsystemen, die ihrerseits mit den N-heterocyclischen Ringen verschmolzen sind, können unsubstituierte Kohlenwasserstoffreste oder übliche funktionelle Substituenten mit der Maßgabe sein, daß sie mit den Glycidylgruppen der erfindungsgemäß eingesetzten Wirkstoffe wenigstens bei Normaltemperatur nicht reaktiv sind. Im folgenden werden eine Reihe spezieller Verbindungen geschildert, die sich der breiten erfindungsgemäßen Definition unterordnen. Im Zusammenhang mit diesen speziellen Verbindungen sind spezifische Aussagen zu Substitutionsmöglichkeiten an den N-heterocyclischen Systemen gemacht. Diese Aussagen haben dabei im Rahmen der Erfindungsbeschreibung allgemeine Gültigkeit, sie sind also nicht nur auf den bestimmten Verbindungstyp beschränkt, bei dessen Erläuterung sie verwendet werden. Die Angaben gelten entsprechend auch für andere Grundkörper, die sich unter der allgemeinen Definition subsummieren lassen.

Eine Ausführungsform der vorliegenden Erfindung betrifft Arzneimittel, die bestimmte mit Glycidylresten substituierte Benzimidazolonderivate enthalten und eine überraschend starke cytostatische Wirksamkeit besitzen.

Gegenstand der Erfindung sind dementsprechend in dieser Ausführungsform Arzneimittelzubereitungen mit cytostatischer Wirksamkeit, enthaltend Verbindungen der allgemeinen Formel II

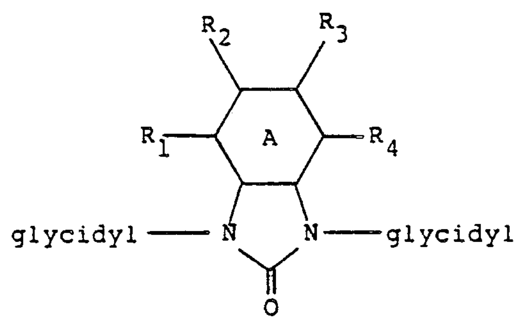

(II)

in der A ein aromatischer, cycloaliphatischer oder olefinisch ungesättigter 6-Ring ist, der auch ein Heteroatom — insbesondere N oder O — enthalten kann, und die Reste $R_1$, $R_2$, $R_3$ und $R_4$ soeiw Glycidyl die folgende Bedeutung haben:

$R_1$ bis $R_4$: gleiche oder verschiedene Reste und dabei Wasserstoff, Halogen oder Kohlenwasserstoffreste mit jeweils nicht mehr als 8 C-Atomen

Glycidyl: ein Rest der allgemeinen Formel

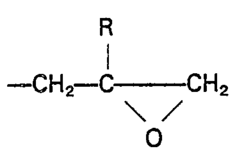

in der R Wasserstoff oder ein Methylrest ist.

Erfindungsgemäß wird damit vorgeschlagen, Diglycidylverbindungen, die Benzimidazolonderivate sind oder letztlich vom Benzimidazolon abgeleitet werden können und sich durch das Vorliegen von 2 Glycidylresten als Substituenten an den Stickstoffatomen des 5-Ringsystems auszeichnen, zum angegebenen Zweck einzusetzen. Eine ganze Reihe von Verbindungen dieser Art sind an sich bekannt. Ihre Herstellung wird beispielsweise in der DE—A—23 00 010 und in der NL—A—73 00 191 beschrieben, wobei in diesen Druckschriften verschiedene Herstellungs-möglichkeiten genannt sind. Ein besonders einfacher Weg der Herstellung geht von den Benzimidazolongrundkörpern aus, in die dann die Glycidylreste an den beiden N-Atomen des Mehrringsystems eingeführt werden. Die Herstellung einer Vielzahl solcher substituierter Benzimidazolone ist beispielsweise geschildert in J. Am. Chem. Soc. Vol. 80 (1958) 1657—1664.

Diglycidylverbindungen der hier betroffenen Art haben bisher ausschließlich Interesse im technischem Bereich insbesondere im Gebiet der Kunststoffchemie gefunden. Bei ihrem erfindungsgemäßen Einsatz zeigen sie eine überraschend hohe cytostatische Wirksamkeit, die in Tierversuchen ermittelt werden konnte.

Der Einfachheit halber werden die im Rahmen der Erfindung eingesetzten Diglycidylverbindungen im

folgenden als Benzamidazolon-Derivate bezeichnet, wenn auch solche Abkömmlinge in diesen Begriff eingeschlossen sind, in denen der 6-Ring A aus der allgemeinen Formel II teilweise oder vollständig gesättigt ist.

Das Ringsystem A der allgemeinen Formel II kann in den Positionen $R_1$ bis $R_4$ unsubstituiert sein, so daß hier diese Reste Wasserstoff bedeuten. Einer oder mehrere dieser Reste können jedoch auch Halogen und/oder Kohlenwasserstoffreste sein. Beim Vorligen mehrerer Substituenten können dabei gleiche oder verschiedene Substituenten zugegen sein. Als Halogen besonders bevorzugt sind Chlor und Brom, Jod und Fluor sind jedoch nicht ausgeschlossen.

Bedeutet wenigstens einer der Reste $R_1$ bis $R_4$ einen Kohlenwasserstoffrest, so soll dieser nicht über 8 C-Atomen aufweisen. Interessant können insbesondere Reste sein, die bis zu 6 oder vorzugsweise sogar nur bis zu 4 C-Atome enthalten. Methyl- und/oder Ethylresten kann neben Wasserstoff besondere Bedeutung zukommen.

Als Kohlenwasserstoffreste kommen geradkettige und/oder verzweigte Alkyl- und Alkenylreste der angegebenen Kohlenstoffzahl in Betracht. Geeignet sind weiterhin Arylreste. Im Sinne der erfindungsgemäßen Definition fallen in diese Gruppe aromatische Reste im engeren Sinne, die also ausschließlich aus dem Rest eines aromatjschen Ringsystems bestehen, wie auch aromatische Reste enthaltende Substituenten von der Art der Alkarylreste beziehungsweise Aralkylreste. Bedeutet wenigstens einer dieser Reste $R_1$ bis $R_4$ einen solchen Arylrest, so sind hier insbesondere 1-kernige Substituenten bevorzugt. Typische Vertreter sind Phenyl, Benzyl, Tolyl, Xylyl und verwandte Verbindungen, der angegebenen Kohlenstoffanzahl.

Wenigstens einer der Substituenten $R_1$ bis $R_4$ kann weiterhin einen Cycloalkyl- oder Cycloalkenylrest bedeuten. Auch hier gilt, daß dieser Begriff Substituenten einschließt, die einen entsprechenden Cycloalkyl- beziehungsweise Cycloalkenylbestandteil aufweisen, wie es sinngemäß zuvor für den Begriff der Arylreste erläutert wurde. Auch im Falle der gesättigten oder teilgesättigten ringförmigen Substituenten sind die einkernigen Ringsysteme auf Basis von Cyclopentyl, Cyclohexyl und ihren Abkömmlingen bevorzugt.

In einer bevorzugten Ausführungsform der Erfindung wiesen die Substituenten $R_1$ bis $R_4$ in der Summe nicht mehr als 12 Kohlenstoffatome auf, wobei bevorzugt nicht mehr als 10 Kohlenstoffatome vorliegen. Insbesondere beträgt in einer bevorzugtgen Ausführungsform die Summe der Kohlenstoffatome dieser Substituenten nicht mehr als 8 insbesondere nicht mehr als 4 C-Atome.

Bevorzugte Bedeutungen für $R_1$ bis $R_4$ sind Wasserstoff, $C_1$—$C_4$-Alkyl, und/oder Halogen. Bevorzugt ist weiterhin, daß wenigstens einer, insbesondere 2 oder 3 dieser Reste Wasserstoff bedeuten.

Besonders bevorzugt werden die N,N'-Digylcidylbenzimidazolone gemäß Formel II mit folgender Bedeutung für $R_1$ bis $R_4$.

| $R_1$ | $R_2$ | $R_3$ | $R_4$ |
|---|---|---|---|
| Methyl | — | — | — |
| Methyl | — | Methyl | — |
| — | Methyl | Methyl | — |
| Methyl | Methyl | Methyl | Methyl |
| — | Ethyl | — | — |
| Ethyl | — | — | — |
| Isopropyl | — | — | — |
| — | n-Propyl | — | — |
| — | i-Propyl | — | — |
| — | n-Butyl | — | — |
| — | 5-Butyl | — | — |
| — | n-Capryl | — | — |
| — | Acetyl | — | — |
| — | Capronyl | — | — |
| — | Hydroxyl | — | — |
| — | Methoxy | — | — |
| — | — | — | — |
| — | — | — | — |
| — | Fluor | — | — |
| — | Brom | — | — |
| Chlor | — | Chlor | — |
| Chlor | — | — | — |
| — | Chlor | Chlor | — |
| Chlor | Chlor | Chlor | — |
| — | Ureido | — | — |
| — | — | — | — |

Gegenstand der vorliegenden Erfindung sind weiterhin Arzneimittelzubereitungen mit cytostatischer Wirksamkeit, enthaltend Verbindungen der allgemeinen Formel III

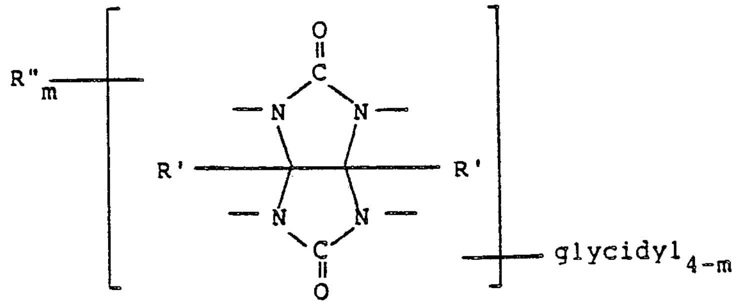

(III)

in der R', R'', Glycidyl und m die folgende Bedeutung haben:

R': gleiche oder verschiedene Reste aus der Gruppe Wasserstoff, Kohlenwasserstoffreste mit vorzugsweise bis zu 8 Kohlenstoffatomen,

R'': gleiche oder verschiedene Reste aus der Gruppe Wasserstoff, Kohlenwasserstoffreste mit vorzugsweise bis zu 4 Kohlenstoffatomen,

m: eine Zahl von 0, 1 oder 2 sowie

Glycidyl: ein Rest der allgemeinen Formel

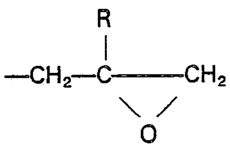

in der R Wasserstoff oder ein Methylrest ist.

Die erfindungsgemäß ausgewählten Glykoluril-Derivate zeichnen sich dadurch aus, daß an dem bicyclischen System wenigstens zwei Glycidylreste als Substituenten an den Stickstoffatomen des Ringsystems vorliegen. Es ist dabei besonders bevorzugt, Verbindungen der allgemeinen Formel III einzusetzen, in denen zwei Glycidylreste in N-Substitution vorgesehen sind, wobei diese beiden Glycidylreste am gleichen 5-Ring (1,3-Stellung) angreifen. Insbesondere fallen aber auch in den erfindungsgemäß bevorzugten Bereich der Verbindung der allgemeinen Formel III die Glycidylderivate, bei denen die beiden Glycidylgruppen auf die beiden 5-Ringsysteme verteilt sind (1,5- bzw. 1,7-Stellung). Auch das Vorliegen von drei Glycidylgruppen (1,3,5-bzw. 1,3,7-Stellung) kann zu erfindungsgemäß bevorzugt einzusetzenden Verbindungen führen.

Die Reste R' in den Verbindungen der allgemeinen Formel III sind insbesondere Wasserstoff, beliebige Kohlenwasserstoffreste mit vorzugsweise jeweils nicht mehr als 8 Kohlenstoffatomen. Es kann zweckmäßig sein, Kohlenwasserstoffreste mit nocht stärker begrenzter C-Zahl einzusetzen, so daß die obere Kohlenstoffgrenze jedes einzelnen dieser Reste R' beispielsweise bei 4 C-Atomen liegen kann.

Als Kohlenwasserstoffreste kommen im hier betroffenen Fall (R') geradkettige und/oder verzweigte Alkyl- und Alkenylreste der angegebenen Kohlenstoffzahl in Betracht. Geeignet sind weiterhin Arylreste. Im Sinne der erfindungsgemäßen Definition fallen in diese Gruppe aromatische Reste im engeren Sinne, die also ausschließlich aus dem Rest eines aromatischen Ringsystems bestehen wie auch aromatische Reste enthaltende Substituenten von der Art der Alkarylreste bzw. Aralkylreste. Bedeutet R' einen Aryl-, Aralkyl- oder Alkarylrest, so sind hier insbesondere 1-kernige Substituenten bevorzugt. Typische Vertreter sind Phenyl, Benzyl, Tolyl, Xylyl und verwandte Verbindungen.

Einer oder beide Reste R' können weiterhin Cycloalkyl- oder Cycloalkenylreste bedeuten. Auch hier gilt, daß dieser Begriff Substituenten einschließt, die einen entsprechenden Cycloalkyl- bzw. Cycloalkenylbestandteil aufweisen, wie es sinngemäß zuvor für den Begriff der Arylreste erläutert wurde. Auch im Falle der gesättigten oder teilgesättigten ringförmigen Substituenten sind die einkernigen Ringsysteme auf Basis von Cyclopentyl, Cyclohexyl und ihren Abkömmlingen bevorzugt.

In einer besonders bevorzugten Ausführungsform der erfindungsgemäß eingesetzten Glykolurilderivate weisen die substituierenden Reste R' zusammen nicht mehr als 12 insbesondere nicht mehr als 10 Kohlenstoffatome auf. Es kann dabei besonders bevorzugt sein, daß die Summe der Kohlenstoffatome in diesen Resten R' nicht mehr als 8 insbesondere nicht mehr als 4 oder höchstens 6 Kohlenstoffatome ausmacht.

Die gegebenenfalls vorliegenden Reste R'' sind — sofern R'' nicht die Bedeutung von Wasserstoff hat — beliebige Kohlenwasserstoffreste die gleich oder verschieden sein können so enthält jeder einzelne dieser Reste nicht mehr als 4 C-Atome.

Als Kohlenwasserstoffreste kommen auch für R'' also beispielsweise geradkettige und/oder verzweigte Alkyl- und Alkenylreste der angegebenen Kohlenstoffzahl in Betracht. Geeignet sind weiterhin Arylreste. Im einzelnen gelten hier die entsprechenden Angaben zu den Resten R', so daß also zu den Arylresten auch Alkaryl- bzw. Aralkylreste zählen. Auch hier sind einkernige Substituenten bevorzugt. Sinngemäß gelten

weiterhin die im Zusammenhang mit der Definition von R′ gemachten Angaben zu Cycloalkyl- oder Cycloalkenylresten sowie zu heterocyclischen Resten.

Besonders bevorzugte Verbindungen gemäß Formel III sind die N,N′-Diglycidylglykolurile mit folgenden Resten R′ und R″:

| R″ | Anzahl R″ | R′ | Anzahl R′ |
|---|---|---|---|
| Methyl- | 2 | — | — |
| — | — | Methyl- | 2 |
| — | — | Methyl- | 1 |
| Methyl- | 2 | Methyl- | 2 |
| Methyl- | 2 | Phenyl- | 2 |
| — | — | Phenyl- | 2 |
| Methyl- | 1 | — | — |

In einer weiteren Ausführungsform sind Gegenstand der vorliegenden Erfindung Arzneimittelzubereitungen mit cytostatischer Wirksamkeit enthaltend Verbindungen der allgemeinen Formel IV

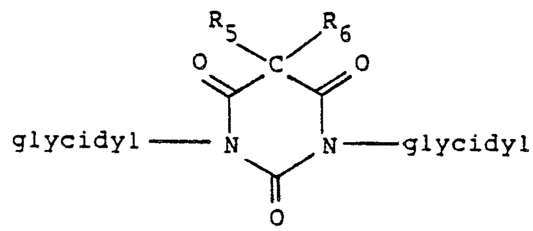

(IV)

in der die Reste $R_5$, $R_6$ und Glycidyl die folgende Bedeutung haben:

$R_5$ und $R_6$:

(1) gleiche oder verschiedene Reste aus der Gruppe Wasserstoff und Kohlenwasserstoffreste mit jeweils bis zu 12 C-Atomen.

(2) $R_5$ und $R_6$ sind gemeinsam zu einem Ringsystem geschlossen

Glycidyl: ein Rest der allgemeinen Formel

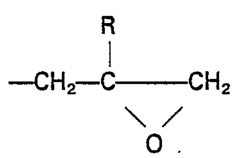

in der R Wasserstoff oder einen Methylrest bedeutet.

Formal ist diese Verbindung die Formel IV, die bestimmte mit Glycidylresten substituierte Barbitursäurederivate darstellt.

Gegenstand der vorliegenden Erfindung sind weiterhin Arzneimittelzubereitungen mit cytostatischer Wirksamkeit, enthaltend Verbindungen der Allgemeinen Formel V

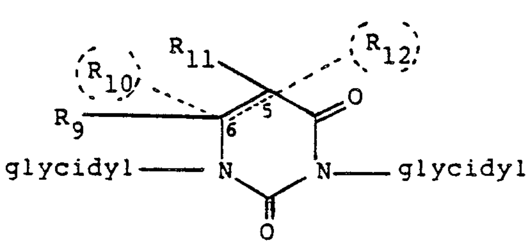

(V)

in der im Fall einer Doppelbindung in 5/6-Stellung die Reste $R_2$ und $R_4$ entfallen und die Reste $R_9$, $R_{10}$, $R_{11}$, $R_{12}$ und Glycidyl die folgende Bedeutung haben:

$R_9$, $R_{10}$, $R_{11}$ und $R_{12}$:

(1) gleiche oder verschiedene Reste aus der Gruppe Wasserstoff, Halogen und Kohlenwasserstoffreste mit jeweils bis zu 12 C-Atomen, die auch substituiert sein können

(2) wenigstens zwei der Reste $R_9$ bis $R_{12}$ sind gemeinsam zu einem Ringsystem geschlossen

(3) wenigstens eines der Paare $R_9/R_{10}$ bzw. $R_{11}/R_{12}$ bilden zusammen den Rest

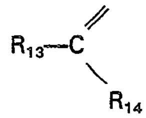

worin $R_{13}$ und $R_{14}$ die Bedeutung von $R_9$ und $R_{10}$ bzw. $R_{11}$ und $R_{12}$ zu (1) oder (2) haben, Erfindungsgemäß wird damit vorgesechlagen, einkernige oder zweikernige Uracil- bzw. Dihydrouracilverbindungen zum angegebenen Zweck einzusetzen, die sich durch das Vorliegen von zwei Glycidylresten als Substituenten an den Stickstoffatomen des bzw. der Ringsystem(e) auszeichnen.

Je nach der Bedeutung der Reste $R_9$ bis $R_{12}$ und Glycidyl in den Verbindungen der allgemeinen Formel VI können verschiedene Klassen von Uracil- bzw. Dihydrouracilverbindungen unterschieden werden:

Enthält der Ring zwei Glycidylreste, dann liegen einkernige Glycidylverbindungen vor, die ihrerseits je nach der Bedeutung der Reste $R_9$ bis $R_{12}$ in verschiedene Untergruppen unterteilt werden können.

In einer ersten Untergruppe sind $R_9$ bis $R_{12}$ Wasserstoff, Halogen oder beliebige Kohlenwasserstoffreste, wobei $R_9$ bis $R_{12}$ im Rahmen dieser Definitiion gleiche oder verschiedene Bedeutung haben können. Liegen Kohlenwasserstoffreste vor, so enthalten diese jeweils bis zu 12 C-Atomen.

Grundsätzlich kommen alle Halogene in Betracht. Besonders geeignet können Chlor, Brom und Fluor sein, die insbesonder in 5-Stellung bedeutsam sein können.

Für die Bedeutung von $R_9$ bis $R_{12}$ als Kohlenwasserstoffreste haben die zuvor angegebenen struktur- und zahlen-mäßigen Aussagen und Begrenzungen volle und uneingeschränkte Gültigkeit wie sie im Zusammenhang der Erläuterungen von $R_5$ und $R_6$ (Formel IV) bzw. zu $R_1$ bis $R_4$ (Formel II) gemacht worden sind. Einfachheitshalber wird darauf verwiesen. Liegen räumlich sperrige Substituenten in 5- und/oder 6-Stellung vor, so sind ein oder bestenfalls zwei solcher Substituenten bevorzugt.

In einer besonders bevorzugten Ausführungsform der hier betroffenen Unterklasse der erfindungsgemäß eingesetzten Uracilderivate weisen die substituierenden Reste $R_9$ bis $R_{12}$ — zusammen nicht mehr als 12 Kohlenstoffatome auf. Es kann dabei besonders bevorzugt sein, daß die Summe der Kohlenstoffatome in diesen Resten $R_9$ bis $R_{12}$ nicht mehr als 10, inbesondere nicht mehr als 8 Kohlenstoffatome ausmacht. Gerade auch für entsprechende Uracilderivate mit nicht mehr als 4 C-Atomen in der Summe dieser Reste $R_9$ bis $R_{12}$ ist eine hohe cytostatische Wirksamkeit nachgewiesen worden.

In einer weiteren Untergruppe der erfindungsgemäß eingesetzten Verbindungen der allgemeinen Formel VI sind wenigstens 2 der Substituenten $R_9$ bis $R_{12}$ zusammen mit dem von ihnen substituierten Kohlenstoffatom(en) des Uracilrings zu einem (oder mehreren) cyclischen Ringsystem(en) geschlossen. Solche Ringsysteme können gesättigter oder ungesättigter isocyclischer oder heterocyclischer Natur sein.

Wie zuvor angegeben, kennzeichnet sich eine weiter Untergruppe der Verbindungen der allgemeinen Formel V für den erfindungsgemäßen Zweck dadurch, daß — im Fall der Dihydrouracilverbindungen — wenigstens eines der Paare $R_9/R_{10}$ und $R_{11}/R_{12}$ gemeinsam den Rest

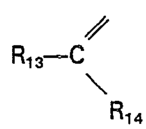

bilden. In diesem Fall haben die Reste $R_{13}$ und $R_{14}$ die bisher angegebene Bedeutung von $R_9$ bis $R_{12}$. Bevorzugt sind hier Verbindungen, in denen nur eines der Paare $R_9/R_{10}$ bzw. $R_{11}/R_{12}$ einen solchen Rest mit $R_{13}$ und $R_{14}$ bilden.

Gegenstand der vorliegenden Erfindung sind weiterhin Arzneimittelzubereitungen mit cytostatischer Wirksamkeit enthaltend Verbindungen der allgemeinen Formel VI

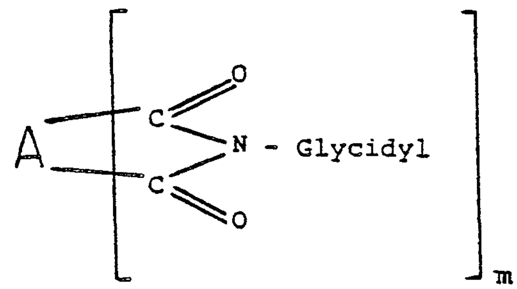

(VI)

in der A ein organischer Rest ist, der auch Heteroatome enthalten kann, m eine Zahl von 2 bis 4 bedeutet und die substituierten Imidgruppierungen

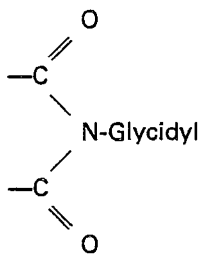

jeweils Imidringen mit 4 bis 10 Ringgliedern angehören und wobei der Glycidylrest die früher gegebene Bedeutung hat.

Erfindungsgemäß wird damit in dieser Ausführungsform vorgeschlagen, Polycarbonsäureimide mit wenigstens 2 cyclischen Imidgruppen und jeweils einem Glycidylrest am Stickstoffatom zum angegebenen Zweck einzusetzen. Es hat sich überraschenderweise gezeigt, daß solche Verbindungen eine bemerkenswert hohe cytostatische Aktivität aufweisen können. So zeigt das N,N'-Diglycidyl-pyromellithsäurediimid (1,2,4,5-Benzoltetracarbonsäurediimid) mit anhängenden Glycidylresten bereits bei sehr niedriger Konzentration eine bemerkenswert hohe cytostatische Aktivität. Die T/C-Raten am P 338 Tumor unter Standard-Prüfbedingungen (9 Injektionen) sind

| | |
|---|---|
| 12,5 mg/kg | 200% |
| 6,25 mg/kg | 156% |

Die erfindungsgemäß geschilderten und als Cytostatika eingesetzten Polycarbonsäureimide enthalten wenigstens 2 mit Glycidylresten substituierte cyclische Imidgruppen der angegebenen Art, es kann aber auch eine größere Anzahl solcher cyclischer Imidgruppen vorliegen. Zweckmäßigerweise liegen nicht mehr als 4 solcher Imidgruppen im Molekül vor, wobei Verbindungen mit 2 oder 3 solcher Imidgruppen besonders bevorzugt sind.

Die Imidgruppen selber bilden mit dem Bestandteil A aus der allgemeinen Formel VI Imidringe mit jeweils wenigstens 4 Ringgliedern und vorzugsweise mit nicht mehr als 10 insbesondere nicht mehr als 8 Ringgliedern. Besondere Bedeutung können erfindungsgemäß solche Verbindungen der allgemeinen Formel VI aufweisen, in denen diese Imidringe 4 bis 7 und insbesondere 5 oder 6 Ringglieder aufweisen. In der Verbindung der allgemeinen Formel VI können die darin vorliegenden Imidringe die gleiche Ringgliederzahl oder auch eine unterschiedliche Ringgliederzahl besitzen. Es ist also möglich, daß 2 fünfgliedrige Glycidyl substituierte Imidringe vorliegen, ebenso können aber beispielsweise auch fünfgliedrige neben sechsgliedrigen Imidringen in einer Verbindung zugegen sein. Die zum jeweiligen Imidringschluß benötigten Glieder aus dem Bestandteil A der Verbindungen der allgemeinen Formel VII können dabei aus unterschiedlichen Bereichen des Bestandteils A stammen, wie es beim eingangs genannten Diglycidyl-pyromellithsäurediimid der Fall ist, es ist aber auch möglich, daß A mit den gleichen Anteilen die Imidringe jeweils vervollständigt, wie es beispielsweise im Falle des N,N'-Diglycidyl-1,1,2,2-ethantetracarbonsäurediimids der Fall ist. Auch eine Vermischung beider Prinzipien — d.h. eine Teilüberschneidung bezüglich der zum jeweiligen Imidringschluß benötigten Glieder des Bestandteils A ist möglich.

Der Bestandteil A des Formelbilds VI ist ganz allgemein ein organischer Rest, der auch Hetero-Atome enthalter kann. Als Heteroatome kommen insbesondere Stickstoff, Sauerstoff in Betracht, wobei diese Heteroatome je nach Beschaffenheit des Restes A einfach oder auch mehrfach auftreten können.

Der Verbindungsbestandteil A kann offenkettig oder ringförmig sein. Im Falle der offenkettigen Struktur kann es sich um eine geradkettige oder eine verzweigte Struktur handeln — unbeschadet der Tatsache, daß Anteile einer solchen geradkettigen Struktur mit den anhängenden substituierten Carbonsäureimidgruppierungen die zuvor dargestellten Ringe ausbilden.

Sowohl die offenkettigen wie die ringförmigen Bauelemente A können gesättigt oder ungesättigt sein. Als ungesättigte Reste kommen insbesondere einfach oder mehrfach olefinisch ungesättigte Bestandteile in Betracht. Bei den ringförmigen Elementen A sind insbesondere auch aromatische Ringe geeignet und gegebenenfalls sogar besonders bevorzugt. Möglich sind auch beliebige Mischstrukturen der hier dargestellten Möglichkeiten, so daß also beispielsweise ringförmige Bauelemente mit offenkettigen verbunden sind, wobei die Glycidyl-substituierten cyclischen Imidgruppen an beliebigen Teilen dieses Bauelements A angreifen können.

Der Bestandteil A ist üblicherweise ein Kohlenwasserstoffrest. Vorzugsweise enthält dieser Rest A bis zu 10 Ketten-bzw. Ringglieder — beispielsweise $CH_2$-Gruppen offenkettiger oder ringförmiger gesättigter oder CH-Gruppen offenkettiger oder ringförmiger ungesättigter bzw. aromatischer Baubestandteile. Der Baubestandteil A ist wenigstens durch ein Glied gekennzeichnet, er kann durch zwei Glieder dargestellt sein und enthält in besonders bevorzugten Fällen bis zu 10 Glieder. Hierbei ist bei den offenkettiven Bestandteilen A jede Zahl dieses Bereichs möglich, während bei ringförmigen Verbindungen 4 Ringglieder die untere Grenze darstellen. Als einfache Ringe sind besonders solche mit 5 oder 6 Ringgliedern bevorzugt. Bei Ringsystemen mit Mehrfachringen können diese Ringe miteinander verschmolzen oder über wenigstens ein gemeinsames Glied miteinander verbunden sein, sie können aber auch als isolierte Ringe nebeneinander vorliegen und lediglich durch eine chemische Bindung oder durch Brückenglieder miteinander verbunden sein.

Der Rest A aus der allgemeinen Formel VII stellt den Rest von Polycarbonsäuren mit wenigstens 4 Carboxylgruppen und insbesondere mit 4 bis 8 Carboxylgruppen dar. Besonders bevorzugt sind Reste von solchen Polycarbonsäuren mit 4 oder 6 Carboxylgruppen. Diese Reste können aliphatischer, cycloaliphatischer oder aromatischer sowie olefinisch ungesättigten Natur sein. Insbesondere sind auch heterocyclische Reste für A geeignet.

Besonders bevorzugt ist ein oberer Grenzwert für das Molekulargewicht dieses Bestandteils A bei etwa 750. In einer weiterhin bevorzugten Ausführungsform der Erfindung liegen die Molekulargewichte jeweils der gesamten Verbindung der allgemeinen Formel VII bei den eingangs genannten oberen Grenzwerten.

Charakteristische Einzelbeispiele für die Struktur des Molekülbestandteils A aus der Formel VI leiten sich im Fall der offenkettigen Bauweise etwa von Polycarbonsäuren der Art der 1,1,2,2-Ethantetracarbonsäure oder der 1,2,3,4 Butantetracarbonsäure ab. Unter Einschluß von Heteroatomen sind Polycarbonsäurereste wie die der Ethylendiamintetraessigsäure geeignet.

Für ringförmige Reste A sei auf die aromatischen Polycarbonsäuren von der Art der Pyromellithsäure bzw. Naphthalintetracarbonsäure verwiesen. Ein anderes Beispiel ist die Benzolhexacarbonsäure, die ihrerseits 3 Imidgruppen ausbilden kann. Auch im Fall dieser ringförmigen Verbindungen können die Carboxylgruppen an einem Ring bzw. einem zusammenhängenden Ringsystem vorliegen, es ist aber ebenso möglich, die Carbonsäuren einzusetzen, die ihrerseits mit einer weiteren Dicarbonsäure beispielsweise über eine jeweils vorliegende zusätzliche funktionelle Gruppe der oben angegebenen Art miteinander verknüpft sind. So ist durch die Verknüpfung von 2 Molekülen der Trimellithsäure über die jeweils dritte Carboxylgruppe eine Tetracarbonsäure zu gewinnen. Die Verknüpfung kann beispielsweise über die Ausbildung von Ester- oder Amidgruppierungen erfolgen. Durch Mitverwendung mehr als 2 funktioneller Verknüpfungselemente — beispielsweise durch Einsatz von Triaminen zusammen mit Trimellithsäureimid können auch mehr als nur 2 solcher Polycarbonsäuresystem zusammengeschlossen werden.

Was hier für die aromatischen Carbonsäuren ausgeführt ist, gilt sinngemäß für entsprechende cycloaliphatische Carbonsäuren bzw. für solche Verbindungen, in denen Heteroatome, insbesondere O oder N, die Funktion von Ringgliedern übernehmen.

Bei den ringförmigen Bausteinen A aus den Verbindungen der allgemeinen Formel VII liegen die imidbildenden Carboxylgruppen vorzugsweise in o- und/oder in m-Stellung vor, so daß sich insgesamt bevorzugt fünfgliedrige und/oder sechsgliedrige Imidringe ausbilden. Die Erfindung ist, wie bereits angegeben, hierauf nicht eingegrenzt, auch viergliedrige Imidringe kommen in Betracht, aber auch höhergliedrige Ringzahlen beispielsweise solche mit 7 bis 10 Ringgliedern können zweckmäßig sein.

Zahlreiche Polyimide bzw. die ihnen zugrunde liegenden Carbonsäuren der den erfindungsgemäßen Verbindungen der allgemeinen Formel VI zugrunde liegenden Art sind literaturbekannt, genannt seien beispielsweise neben dem einfangs genannten Pyromellithsäurediimid die folgenden Verbindungen, die hier bereits als Glycidyl-substituierte Derivate angegeben sind:

N,N'-Diglycidyl-1,2,3,4-benzoltetracarbonsäurediimid
N,N'-Diglycidyl-1,4,5,8-naphthalintetracarbonsäurediimid
N,N'-Diglycidyl-2,3,4,5-pyridintetracarbonsäurediimid
N,N'-Diglycidyl-2,3,5,6-pyridintetracarbonsäurediimid
N,N'-Diglycidyl-1,2,4,5-cyclohexantetracarbonsäurediimid
N,N'-Diglycidyl-1,1,2,2-ethantetracarbonsäurediimid
N,N'-Diglycidyl-1,2,3,4-butantetracarbonsäuretriimid
N,N',N''-Triglycidyl-benzolhexacarbonsäuretriimid
N,N'-Diglycidyl-tetrahydrofurantetracarbonsäurediimid.

Die Herstellung der erfindungsgemäß eingesetzten Wirkstoffe kann in an sich bekannter Weise erfolgen. Sie gelingt im allgemeinen durch Einführung der Glycidylgruppen in die N-Substitution. Hierzu

wird in an sich bekannter Weise zunächst das heterocyclische ein- oder mehrkernige Molekül des jeweils betroffenen Wirkstoffs hergestellt, wobei jedoch zunächst anstelle der N-Glycidyl-Gruppierung eine —NH-Gruppierung vorliegt. Abschließend erfolgt dann der Ersatz dieses Wasserstoffs am Stickstoff durch die Glycidylgruppe.

Für diese abschließende Reaktionsstufe bestehen vor allem zwei grundsätzliche Möglichkeiten. Die eine liegt in der direkten Einführung der Glycidyl-Gruppierung durch Umsetzung der NH-Gruppierung mit Epihalohydrinen, insbesondere Epichlorhydrin oder Epibromhydrin. Der andere Weg vervollständigt den Molekülaufbau in zwei Reaktionsschritten. Es werden zunächst die entsprechenden allylsubstituierten Vorprodukte gebildet, woraufhin in einer abschließenden Verfahrensstufe die Allylgruppe epoxidiert wird.

Zur Umsetzung von —NH-Gruppen mit Epihalohydrinen besteht eine umfangreiche Literatur. Die Umsetzung kann in Gegenwart einer geringen Menge einer quartären Ammoniumverbindung als Katalysator durchgeführt werden (vgl. hierzu beispielsweise Houben-Weyl, "Methoden der organischen Chemie" Band 14/2 (1963), 497, 547). Die Umsetzung von Allylhalogeniden mit NH-Gruppierungen der hier betroffenen Art ist beispielsweise beschrieben in der US—A—3 376 301.

Die Epoxidation einer primär gebildeten Allylgruppe kann in an sich bekannter Weise mit Persäure vorgenommen werden. Die Epoxidation von Allylisocyanuaraten mit Persäuren ist z. B. in Houben-Weyl aaO, Band 6/3, 385 ff. beschrieben. Sie kann beispielsweise in Gegenwart einer geringen Menge einer quartären Ammoniumverbindung als Katalysator durchgeführt werden.

Die Umsetzungen der gebildeten Vorstufen mit Epihalohydrinen bzw. Allylhalogeniden erfolgt zweckmäßigerweise im Temperaturbereich von etwa 50 bis 150°C, vorzugsweise von etwa 70 bis etwa 125°C. Allylhalogenid bzw. Epihalohydrin wird in einem Molverhältnis zur eingesetzten —NH—CO-Verbindung von wenigstens 2:1 eingesetzt, wobei aber auch mit beträchtlichem Überschuß gearbeitet werden kann, beispielsweise bis zu einem Molverhältnis von 10:1. Das Arbeiten mit Molverhältnissen im Bereich von 2 bis 4 Mol Allylhalogenid bzw. Epihalohydrin je Mol —NHCO-Ausgangsverbindung kann besonders zweckmäßig sein. Die bevorzugten Allylhalogenide bzw. Epihalohydrine enthalten Chlor oder gegebenenfalls Brom als Halogen.

Die Reaktion kann in polaren, insbesondere aprotischen Lösungsmitteln vorgenommen werden, die wenigstens einen der Reaktionspartner teilweise lösen und den Reaktanten gegenüber nicht reaktiv sind. Ein besonders zweckmäßiges Lösungsmittel ist die Klasse der Dialkylformamide, insbesondere der niederen Dialkylformamide wie Dimethylformamid. Die bevorzugte Reaktionszeit beträgt 1 bis 10 Stunden, insbesondere 2 bis 5 Stunden.

Auch die Epoxidation der Allylgruppierungen mittels Persäuren wird vorzugsweise in Lösungsmitteln durchgeführt Geeignet sind auch hier polare Lösungsmittel, beispielsweise Halogenkohlenwasserstoffe oder Alkohole. Die geeignete Reaktionstemperatur liegt üblicherweise im Bereich von 0 bis 50°C, insbesondere zwischen etwa 10 und 30°C. Die Persäure wird zweckmäßigerweise in annährend äquivalenter Menge oder nur in leichtem Überschuß eingesetzt.

m-Chlorperbenzoesäure ist als Handelsprodukt leicht zugänglich und für die Durchführung der Reaktion geeignet. Die Reaktionsdauer liegt in der Regel im Bereich von 24 Stunden oder mehr, beispielsweise bis zu 48 Stunden.

Angaben zur Herstellung von Verbindungen der allgemeinen Formel II sind bereits im Zusammenhang mit ihrer Schilderung gemacht worden. Verwiesen wird nochmals auf die DE—A—23 00 010 und die NL—A—73 00 191.

Die Herstellung der erfindungsgemäß einzusetzenden Verbindungen der allgemeinen Formel III kann beispielsweise auf folgendem Wege erfolgen, wobei allerdings auch andere Wege prinzipiell gangbar sind: wie in der DE—A—19 32 306 beschrieben, werden die in gewünschter Weise substituierten dicyclischen Glycoluril-Ausgangsverbindungen mit Epihalohydrinen unter Abspaltung von Halogenwasserstoff mit Alkali umgesetzt.

1. Umsetzung der 1,3-unsubstituierten Hydantoine bzw. Hydantoinderivate mit einer Halogenhydrinverbindung, insbesondere Epichlorhydrin oder Epibromhydrin unter Abspaltung von Halogenwasserstoff mit Alkalien. Einzelheiten dieses Verfahrenstyps sind in der DE—A—19 12 281 am Beispiel des 5,5-Dimethylhydantoins beschrieben.

2. Epoxydierung der N-Allyl-substituierten Hydantoine bzw. Hydantoinderivate. Diese acylsubstituierten Hydantoinausgangsverbindungen sind ihrerseits beispielsweise durch Umsetzung von 1,3-unsubstituierten Hydantoinen bzw. Hydantoinderivaten mit Allylhalogenid herstellbar. Ein solches Herstellungsverfahren ist in seinen Prinzipien beispielsweise in der DE—OS 21 32 988 beschrieben allerdings nicht für Hydantoinverbindungen, sondern für den Fall des Triglycidylisocyanurats.

Zur einschlägigen Literatur wird verwiesen auf E. H. Catsiff, RE. Coulehan et al. Am. Chem. Soc. Div. of Org. Coat. and Plast. Chem. Pap. *39* (1978) S. 139—145 sowie auf die DE—A—21 25 355 und DE—A—27 27 266 sowie die US—A—4 125 516.

Die hier für einkernige Hydantoinderivate gemachten Angaben zur Herstellung gelten sinngemäß auch für die Herstellung der zweikernigen Hydantoinverbindungen der allgemeinen Formel IV.

Analog anwendbar sind die Herstellungsangaben zur Gewinnung der erfindungsgemäß eingesetzten Barbitursäureverbindungen der allgemeinen Formel V.

Die erfindungsgemäßen Arzneimittel können einzelne definierte Verbindungen gemäß der Erfindung und insbesondere der angegebenen Formeln II bis X enthalten, es hat sich aber gezeigt, daß insbesondere

auch Wirkstoffgemische mehrerer Verbindungen hochwirksame Cytostatika sind. Im Rahmen der Erfindung kann es weiterhin zweckmäßig sein, einzelne bestimmte oder eine Mischung mehrerer Verbindungen der erfindungsgemäßen Definitionen in Abmischung mit den TGI-Verbindungen gemäß der erwähnten älteren Schutzrechte zu verwenden. Auch eine Kombinationstherapie in Verbindung mit anderen Cytostatika, wie Derivaten des Stickstofflost oder Fluoruracil ist möglich.

Ganz allgemein gilt für die im Rahmen der Erfindung eingesetzten Verbindungen der angegebenen allgemeinen Formeln, daß die neben den Glycidylgruppen gegebenenfalls vorliegenden Reste bzw. Substituenten wenigstens unter Normalbedingungen keine oder keine wesentliche Reaktivität mit den Epoxidgruppen der Glycidylsubstituenten zeigen bzw. zeigen sollen. Auf diese Weise ist sichergestellt, daß die erfindungsgemäß verwendeten Wirkstoffe hinreichend lagerbeständig sind und keine unerwünschte Umsetzung unter Vernichtung der Epoxidgruppierungen stattfindet.

Die erfindungsgemäß eingesetzten Glycidyl-substituierten heterocyclischen Verbindungen treten üblicherweise in verschiedenen stereoisomeren Formen auf. Grundsätzlich eignen sich alle diese verschiedenen Formen für die Zwecke der Erfindung. Sie können dabei in Mischung oder auch in Form bestimmter isolierter Isomeren eingesetzt werden.

Zur Verwendung als Cancerostatika sollten die Wirksubstanzen mittels geeigneter Vehikel appliziert werden. Hier eignen sich die üblichen Hilfs- bzw. Trägerstoffe für pharmakologische Zubereitungen. Häufig hat sich hier die Verwendung von wäßrigen Systemen gegebenenfalls mit verträglichen Glycolethern wie Glycolmonoethylethern oder Butylenglycolmethylether oder Propylenglycolmethylether bewährt, insbesondere wenn der Wirkstoff parenteral appliziert werden soll. Bei oraler Verabreichung sind die pharmazeutisch üblichen Hilfs- bzw. Trägerstoffe anwendbar, sofern sie eine entsprechende Verträglichkeit mit den Glycidylverbindungen aufweisen.

Im Tierexperiment hat sich die Verwendung von frisch hergestellten wäßrigen Lösungen, die i.p. gegeben werden, als zweckmäßig erwiesen.

Die erfindungsgemäß eingesetzten Verbindungen sind wirksam gegen verschiedene Leukämieformen sowie maligne Neoplasmen wie Lungencarcinom, Coloncarcinom, Melanome, Ependymoblastom und Sarcome. In einigen Fällen konnte eine deutliche Überlegenheit gegenüber bekannten und auf dem Markt befindlichen cytostatischen Arzneimitteln festgestellt werden.

Die erfindungsgemäß beschriebenen Polyglycidyl-substituierten Heterocyclen liegen in den Arzneimittelgemischen gemäß der Erfindung üblicherweise in Konzentrationen bis zu etwa 10 Gewichtsprozent — bezogen auf die Arzneimittelmischung — vor. Geeignet ist beispielsweise der Bereich von 0,05 bis 10 Gewichtsprozent, insbesondere ein Bereich von 0,05 bis 5 Gewichtsprozent.

In den folgenden Beispielen werden sowohl die Herstellung als auch die Anwendung im Rahmen von Tierexperimenten für charakteristische Verbindungen der erfindungsgemäßen Definition beschrieben. Die Prozentangaben beziehen sich jeweils auf Gewichtsprozent, sofern nicht im Einzelfall andere Angaben gemacht sind.

## Beispiel 1

N,N'-Digylcidyl-Benzimidazolon

5 g Benzimidazolon werden mit 200 g Epichlorhydrin unter Zusatz von 50 mg Tetraethylammoniumbromid 4 Stunden am Rückfluß gekocht. Die erkaltete Lösung wird filtriert, mit 40 g $Na_2SO_4$ und 3 g gepulverter NaOH versetzt und 1,5 Stunden bei Raumtemperatur gerührt. Man filtriert ab und dampft im Vakuum zur Trockene ein. Es verbleibt eine braune Masse, die nach einiger Zeit kristallisiert. Umkristallisation aus Methanol ergibt 5 g kristalline Verbindung, Fp. 102°C, Epoxidgehalt 13,7% (Theorie 14,2%). Das Massenspektrum stützt die angegebene Struktur.

## Beispiel 2

Analog zu Beispiel 1 wurden hergestellt

| | |
|---|---|
| N,N'-Diglycidyl-5-acetylbenzimidazolon | EpO: 10,7% (ber. 11,1%)<br>Fp: 127°C<br>C: 62,0% (ber. 62,5%)<br>H: 5,67% (ber. 5,59%)<br>N: 9,65% (ber. 9,72%) |
| N,N'-Diglycidyl-5,6-dimethylbenzimidazolon | EpO: 11,4% (ber. 11,7%)<br>Fp: 135—140°C |
| N,N'-Diglycidyl-5-chlorbenzimidazolon | EpO: 10,9% (ber. 11,3%)<br>Fp: 127—130°C<br>C: 55,2% (ber. 55,3%)<br>H: 4,9% (ber. 5,3%)<br>N: 9,6% (ber. 9,9%) |

| | |
|---|---|
| N,N'-Diglycidyl-5-Nitrobenzimidazolon | EpO: 10,3% (ber. 10,9%)<br>Fp: 150—154°C<br>C: 52,7% (ber. 53,2%)<br>H: 4,6% (ber. 5,1%)<br>N: 13,9% (ber. 14,3%) |
| N,N-Diglycidyl-5,6-dichlor-benzimidazolon | EpO: 9,6% (ber. 10,2%)<br>Fp: 135 bis 140°C |
| N,N-Diglycidyl-5-methoxy-benzimidazolon | EpO: 11,2% (ber. 11,6%)<br>Fp: 60 bis 62°C |
| N,N-Diglycidyl-5-caproyl-benzimidazolon | EpO: 8,64% (ber. 9,25%)<br>Fp: 106 bis 107°C |
| N,N-Diglycidyl-5-capryl-benzimidazolon | EpO: 7,9% (ber. 9,6%)<br>Fp: 51 bis 52°C |

Beispiel 3

Die nachfolgenden Versuche wurden durchgeführt nach Testvorschriften des National Cancer Institute Bethesda, Maryland 200014, veröffentlicht in "Cancer Chemotherapy Reports" Part. 3, September 1972, Vol. 3, Nr. 2. Als Wirksubstanz wurde 1,3-Diglycidyl-benzimidazolon des vorstehenden Beispiels 1 verwendet. Die Substanz wurde als wäßrige, 1%ige Injektionslösung unmittelbar vor der Applikation frisch hergestellt.

Bei Mäusen wurde gemäß Protokoll 1200 (Seite 91, c.) die Tumorart P 388 (Leukämie) i.p. mit $10^6$ Zellen/Maus gesetzt. Die unbehandelten Tiere hatten eine mittlere Überlebensdauer von 9,9 Tagen.

Es wird der Wirkstoff appliziert. Der Wirkstoff wird dabei in 9 Gaben in unterschiedlichen Versuchsreihen einmal zu je 200 mg/kg und in einer anderen zu 100 mg/kg und in einer dritten Versuchsreihe zu je 50 mg/kg verabreicht. In beiden Fällen wird eine signifikante Verlängerung der Lebensdauer der behandelten Testtiere gegenüber der Kontrollgruppe der nicht behandelten Tiere erzielt. Die durchschnittliche Lebensdauer der Testgruppe mit der Verabreichung von je 200 mg/kg beträgt 20 Tage, die Verlängerungsrate T/C beträgt damit 202%. Bei der Testgruppe mit der Dosis von 100 mg/kg beträgt die durchschnittliche Lebenserwartung der Versuchstiere 18 Tage, T/C 181%. Die Gruppe mit der Dosis 50 mg/kg hatte eine durchschnittliche Lebenserwartung von 15,3 Tagen, T/C 154%.

In einem Wiederholungsversuch wurden die Ergebnisse bestätigt.

In gleicher Weise wurden weitere Benzimidazolonderivate ausgeprüft. Die erhaltenen Werte sind in der folgenden Tabelle 1 zusammengefaßt.

TABELLE 1

| Diglycidyl-benzimidazolon-Derivate | | Dosierung mg/kg Maus | Testergebnis in % T/C | |
|---|---|---|---|---|
| $R_2$ | $-H$ | 200 | 202 | 166 |
| $R_3$ | $-H$ | 100 | 181 | 161 |
| | | 50 | 154 | 136 |
| | | 25 | | 133 |
| $R_2$ | $-H$ | 200 | — | — |
| $R_3$ | $-Cl$ | 100 | 234 | 92 |
| | | 50 | 191 | 181 |
| | | 25 | — | 156 |
| | | 12,5 | — | 145 |
| $R_2$ | $-Cl$ | | | |
| $R_3$ | $-Cl$ | 100 | 120 | |
| | | 50 | 158 | |
| $R_2$ | $-H$ | | | |
| $R_3$ | $-O-CH_3$ | 100 | 225 | |
| | | 50 | 175 | |
| $R_2$ | $-H$ | 200 | 130 | |
| $R_3$ | $-\underset{\underset{O}{\|}}{C}-C_5H_{11}$ | 100 | 198 | |
| | | 50 | 167 | |

Beispiel 4

Diglycidyl-butylglykoluril

10 g trockenes N-Butylglykoluril (0,05 Mol) werden mit 0,1 g Tetraethylammoniumbromid und 200 g Epichlorhydrin 4 Stunden im Autoklaven auf 140°C erhitzt. Nach dem Abkühlen läßt man die filtrierte Lösung bei 45 bis 50°C und vermindertem Druck (Wasserstrahlvakuum) invensiv am Rückfluß sieden und tropft 8 g 50%ige wäßrige Natronlauge zu. Das Wasser wird dabei kontinuierlich destillativ entfernt. Das entstandene Kochsalz wird abfiltriert und das Epichlorhydrin abdestilliert. Nach sorgfältiger Entfernung von höhersiedenden Anteilen im Vakuum (0,1 Torr), wobei das Produkt auf nicht mehr als 80°C erhitzt werden sollte, erhält man 9,6 g Diglycidylbutylglykoluril in Form eines hellgelben Harzes; Epoxidgehalt 10,1%, Theorie 10,3%. Das Massenspektrum stützt die angegebene Struktur.

Beispiel 5

3,6 Diglycidyl-1,4-dimethylglykoluril

Aus 30 g 1,4-Dimethylglykoluril, 0,4 g Tetraethylammoniumbromid und 350 g Epichlorhydrin, 28,6 g 50%iger Natronlauge erhält man nach dem obigen Verfahren 34,8 g 3,6-Diglycidyl-1,4-dimethylglykoluril als hellgelbes nicht kristallisierendes Harz. Epoxidgehalt: 11,0%, Theorie: 11,4%. Das Massenspektrum stützt die angegebene Struktur.

Beispiel 6

Die nachfolgenden Versuche wurden durchgeführt nach den Testvorschriften des Beispiels 3. Als Wirksubstanz wurde in einer ersten Versuchsreihe Diglycidyl-butylglykoluril des vorstehenden Beispiels 4 verwendet. Die Substanz wurde als wäßrige, 1%ige Injektionslösung unmittelbar vor der Applikation frisch hergestellt.

Bei Mäusen wurde gemäß Protokoll 1200 (Seite 91.c.) die Tumorart P 388 (Leukämie) i.p. mit $10^6$ Zellen/Maus gesetzt. Die unbehandelten Tiere hatten eine mittlere Überlebensdauer von 9,8 Tagen.

Es wird der Wirkstoff appliziert. Der Wirkstoff wird dabei in 9 Gaben in unterschiedlichen Versuchsreihen einmal zu je 200 mg/kg, sowie zu je 100 mg/kg und in einer dritten Versuchsreihe zu je 50 mg/kg verabreicht. In allen Fällen wird eine signifikante Verlängerung der Lebensdauer der behandelten Testtiere gegenüber der Kontrollgruppe der nicht behandelten Tiere erzielt. Die durchschnittliche Lebensdauer der Testgruppe mit der Verabreichung von je 200 mg/kg beträgt 27 Tage, die Verlängerungsrate T/C beträgt damit 272%. Bei der Testgruppe mit der Dosis von 100 mg/kg beträgt die durchschnittliche Lebenserwartung der Versuchstiere 19,3 Tage, T/C 194%. Bei einer Testgruppe von 6 weiblichen Mäusen überlebt ein Versuchstier die Testdauer von 30 Tagen bei der Verabreichung von 200 mg/kg pro Einzeldosis. Die Testgruppe mit 50 mg/kg pro Einzeldosis überlebte 16,3 Tage T/C 164%.

Zur Austestung des Wirkstoffgemisches gemäß Beispiel 5 werden Versuchstiere in gleicher Weise wie zuvor mit der Tumorart P 388 (Leukämie) versehen. Auch hier werden dann jeweils 9 Einzelgaben des erfindungsgemäßen Wirkstoffgemisches in Dosen von 200 mg/kg, 100 mg/kg und 50 mg/kg verabreicht.

Die mittlere Lebenserwartung der unbehandelten Kontrolltiere ist 9,5. Die Verlängerungsrate T/C beträgt bei 200 mg/kg 190%. Wird das gleiche Wirkstoffgemisch in einer Menge von 100 mg/kg pro Einzeldosis verabreicht, beträgt die mittlere Überlebensdauer der Versuchstiere 15,8 Tage. Das T/C-Verhältnis beträgt 157%. Bei der Verabreichung des Wirkstoffgemisches in einer Einzeldosis von 50 mg/kg beträgt das T/C-Verhältnis 134%.

Beispiel 7

1,3-Diglycidyl-5,5-diethylbarbitursäure

| | | |
|---|---|---|
| 18,4 g | (0,1 Mol) | Diethylbarbitursäure |
| 736 g | (8,0 Mol) | Epichlorhydrin und |
| 0,36 g | | Tetraethylammoniumbromid |

werden unter Rühren 4 Stunden zum Rückfluß erhitzt (120°C). Dabei geht die Barbitursäure nach 20' in Lösung. Dann wird auf ca. 40 bis 50°C abgekühlt und 8,0 g (0,2 Mol) NaOH als 50%ige Lösung innerhalb 20' zugetropft. Gleichzeitig wird durch Destillation im Vakuum das Wasser laufend entfernt. Es wird 1,5 h bei ca. 45°C nachgerührt. Nach Absaugen des Kochsalzes wird in Rotavapor eingeengt und am Hochvakuum getrocknet.

Auswaage: 29,0 g gelb-viskoses Öl

EpO: 10,2% (Th. 10,7%)

Das Massenspektrum stützt die Struktur.

Beispiel 8

1,3-Diglycidyl-5-isobutylbarbitursäure

Aus

| | | |
|---|---|---|
| 18,4 g | (0,1 Mol) | 5-Isobutylbarbitursäure |
| 736 g | (8,0 Mol) | Epichlorhydrin |
| 0,76 g | | Tetraethylammoniumbromit |

Die Herstellung der obigen Verbindung aus den vorstehend aufgeführten Ausgangsmaterialien erfolgte analog Beispiel 10.

Auswaage: 26,2 g gelb-viskoses Öl

EpO: 10,3% (Th. 10,7%)

Das Massenspektrum stützt die Struktur.

Beispiel 9

Die nachfolgenden Versuche wurden durchgeführt nach den Testvorschriften des Beispiels 3. Als Wirksubstanz wurde 1,3-Diglycidyl-5,5-diethylbarbitursäure des vorstehenden Beispiels 10 verwendet. Die Substanz wurde als wäßrige, 1%ige Injektionslösungen unmittelbar vor der Applikation frisch hergestellt.

Bei Mäusen wurde gemäß Protokoll 1200 (Seite 91.c.) die Tumorart P 388 (Leukämie) i.p. mit $10^6$ Zellen/Maus gesetzt. Die unbehandelten Tiere hatten eine mittlere Überlebensdauer von 10,4 Tagen.

Es wird der Wirkstoff appliziert. Der Wirkstoff wird dabei in 9 Gaben in unterschiedlichen Versuchsreihen einmal zu je 100 mg/kg und in einer anderen Versuchsreihe zu je 50 mg/kg verabreicht. In beiden Fällen wird eine signifikante Verlängerung der Lebensdauer der behandelten Testtiere gegenüber der Kontrollgruppe der nicht behandelten Tiere erzielt. Die durchschnittliche Lebensdauer der Testgruppe mit der Verabreichung von je 100 mg/kg beträgt 22 Tage, die Verlängerungsrate T/C beträgt damit 211%. Bei der Testgruppe mit der Dosis von 50 mg/kg beträgt die durchschnittliche Lebenserwartung der Versuchstiere 15,3 Tage, T/C 147%.

In weiteren Versuchsserien werden zusätzliche Testergebnisse — wie in der folgenden Tabelle 3 erfaßt — ermittelt.

TABELLE 3

| Testsubstanz: N,N'-Diglycidyl-barbitursäuren | | Dosierung mg/kg Maus | Testergebnis T/C | |
|---|---|---|---|---|
| $R_5$ | $-CH_2-CH_3$ | 200 | | 230 |
| | | 100 | 211 | 158 |
| $R_6$ | $-CH_2-CH_3$ | 50 | 147 | 111 |
| | | 25 | | 94 |
| $R_5$ | $-H$ | 200 | 194 | 252 |
| $R_6$ | $-CH_2-CH-CH_3$ | 100 | 158 | 184 |
| | $CH_3$ | 50 | 133 | 158 |
| | | 25 | | 129 |

Beispiel 10

Darstellung von N,N'-Diglycidyluracil

11,2 g (0,1 Mol) Uracil, 736 g (8 Mol) Epichlorhydrin und 0,35 g Tetraethylammoniumbromid wurden 4 Stunden zum Rückfluß erhitzt. Hierbei ging das Uracil nach ca. 1 Stunde in Lösung. Das Reaktionsgemisch wurde auf 45 bis 50°C abgekühlt und im Vakuum (15 Torr) intensiv zum Sieden gebracht. Dabei wurden 8 g Natriumhydroxid als 50%ige wäßrige Lösunge innerhalb von 20 Minuten zugetropft. Das Wasser wurde durch azeotrope Destillation laufend entfernt. Nach 2stündigem Rühren bei ca. 40°C wurde entstandenes Kochsalz abgesaugt und Epichlorhydrin abdestilliert. Der erhaltene hellgelbe Sirup wurde im Hochvakuum getrocknet.

Es verblieben 21,5 g eines gelb viskosen Öls.

Epoxidgehalt: 13,4% (Theorie 14,2%)

Das Massenspektrum und das IR-Spektrum stützen die Struktur.

Beispiel 11

Analog der vorstehend gegebenen Vorschrift wurden aus den entsprechenden Ausgangsmaterialien hergestellt:

a) N,N'-Diglycidyl-6-methyluracil
   Ausbeute: 75%, weiße Kristalle
   Fp. 95°C
   Epoxidsauerstoff 12,9% (Theorie 13,4%)
b) N,N'-Diglycidyl-5-methyluracil
   Fp. 87 bis 89°C
   Epoxidsauerstoff 12,82% (Theorie 13,43%)
c) N,N'-Diglycidyl-5-bromuracil
   Fp. 76 bis 79°C
   Epoxidsauerstoff 10,01% (Theorie 10,56%)
d) N,N'-Diglycidyl-5-iodouracil
   schwach-gelber Sirup
   Epoxidsauerstoff 8,66% (Theorie 9,14%)

e) N,N'-Diglycidyl-5-fluoruracil
schwach-gelber Sirup,
Epoxidsauerstoff 12,39% (Theorie 13,21%)

f) N,N-Diglycidyl-1,1'-methylen-bis-uracil
nicht kristallin
EpO: 8,1% (Theorie: 9,2%)

Die Herstellung des 1,1'-Methylen-bis-uracil erfolgt gemäß DE—OS 19 12 291.

Beispiel 12

Die nachfolgenden Versuche wurden durchgeführt nach den Testvorschriften des Beispiels 3. Als Wirksubstanz wurde 1,3-Diglycidyl-uracil aus dem vorstehenden Beispiel 13 verwendet. Die Substanz wurde als wäßrige, 1%ige Injektionslösungen unmittelbar vor der Applikation frisch hergestellt.

Bei Mäusen wurde gemäß Protokoll 1200 (Seite 91.c.) die Tumorart P 388 (Leukämie) i.p. mit $10^6$ Zellen/Maus gesetzt. Die unbehandelten Tiere hatten eine mittlere Überlebensdauer von 10,4 Tagen.

Es wird der Wirkstoff appliziert. Der Wirkstoff wird dabei in 9 Gaben in unterschiedlichen Versuchsreihen einmal zu je 200 mg/kg und in einer anderen Versuchsreihe zu je 100 mg/kg verabreicht. In beiden Fällen wird eine signifikante Verlängerung der Lebensdauer der behandelten Testtiere gegenüber der Kontrollgruppe der nicht behandelten Tiere erzielt. Die durchschnittliche Lebensdauer der Testgruppe mit der Verabreichung von je 200 mg/kg beträgt 24 Tage, die Verlängerungsrate T/C beträgt damit 230%. Bei der Testgruppe mit der Dosis von 100 mg/kg beträgt die durchschnittliche Lebenserwartung der Versuchstiere 16,0 Tage, T/C 153%. In einem Wiederholungsversuch wurden die Ergebnisse bestätigt. Hier lagen die Zahlenwerte T/C bei 198% und 152%.

Weitere Testergebnisse mit Uracilverbindungen sind in der folgenden Tabelle 4 zusammengefaßt.

TABELLE 4

| Testsubstanz | Dosierung mg/kg Maus | Testergebnis T/C | |
|---|---|---|---|
| Beispiel 13 | 200 | 230 | 198 |
| | 100 | 156 | 152 |
| | 50 | 131 | 124 |
| | 25 | | 114 |
| Beispiel 14 | 200 | 189 | |
| | 100 | 146 | |
| | 50 | 103 | |
| | 25 | | |
| Diglycidyl-5-floururacil | 100 | 300 | |
| | 50 | 179 | |
| Diglycidyl-5-joduracil | 200 | 189 | |
| | 100 | 166 | |
| | 50 | 124 | |
| Diglycidyl-5-bromuracil | 100 | 193 | |
| 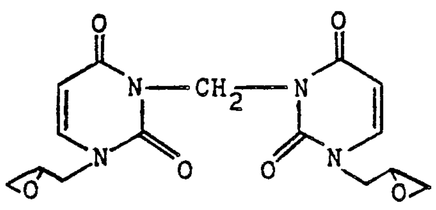 | 50 | 173 | |
| | 200 | 186 | |
| | 100 | 139 | |
| | 50 | 115 | |

Beispiel 13

Die Herstellung von N,N'-Diglycidylbenzoltetracarbonsäure(1,2,4,5,)diimid erfolgte gemäß FR—A—15 59 450 in nachstehend ausführlich beschriebener Weise.

In einem 2 l Dreihalskolben, der mit Rührer, Thermometer und Rückflußkühler versehen war, wurden 108 g Pyromellithsäurediimid, 925 g Epichlorhydrin und 1 g Benzyltrimethylammoniumchlorid gegeben. Die Mischung wurde unter Rühren 3 Stunden am Rückfluß erhitzt. Anschließend wurden 500 g Epichlorhydrin abdestilliert und die Lösung auf 2°C abgekühlt. Das ausgefallene Kristallisat wurde abfilriert und zweimal mit wenig Äthanol gewaschen und getrocknet. Durch Einengen der Mutterlauge auf etwa 150 ccm und Zusatz von 300 ccm Äthanol wurde ein weiteres Kristallisat erhalten. Die Ausbeute betrug insgesamt 118 g N,N'-Diglycidylbenzoltetracarbonsäure(1,2,4,5)diimid.

Durch einmaliges Umkristallisieren aus einem Gemisch aus gleichen Teilen Epichlorhydrin und Äthanol wurde ein Produkt erhalten, das einen Schmelzpunkt von 215 bis 217°C aufwies. Der Epoxidsauerstoffgehalt betrug 7,8% (Methode nach Grennlee).

Beispiel 14

Die nachfolgenden Versuche wurden durchgeführt nach den Testvorschriften des Beispiels 3. Als Wirksubstanz wurde das N,N'-Diglycidylbenzoltetracarbonsäure(1,2,4,5)diimid des Beispiels 16 verwendet. Die Substanz wurde als 1%ige Injektionslösung in Ethylenglycolmonomethylether unmittelbar vor der Applikation frisch hergestellt.

Bei Mäusen wurde gemäß Protokoll 1200 (Seite 91.c.) die Tumorart P 388 (Leukämie) i.p. mit $10^6$ Zellen/Maus gesetzt.

Die mittlere Überlebensdauer der nicht mit dem erfindungsgemäßen Wirkstoff behandelten Tiere wurde bestimmt.

Einer weiteren Gruppe von Versuchstieren wird der Wirkstoff appliziert. Der Wirkstoff wird dabei in neun Gaben in unterschiedlichen Versuchsreihen einmal zu je 12,5 mg/kg und in einer weiteren Versuchsreihe zu je 6,25 mg/kg verabreicht. In allen Fällen wird eine signifikante Verlängerung der Lebensdauer der behandelten Testtiere gegenüber der Kontrollgruppe der nicht behandelten Tiere erzielt. Die Verlängerungsrate T/C beträgt bei der Verabreichung von 12,5 mg/kg 200%, bei der Verabreichung von 6,25 mg/kg 156%.

**Patentansprüche für die Vertragsstaaten: DE CH DE FR GB IT LI NL SE**

1. Arzneimittelzubereitungen mit cytostatischer Wirksamkeit auf Basis von Polyglycidyl-substituierten N-heterocyclischen organischen Ringverbindungen, dadurch gekennzeichnet, daß sie in einem heterocyclischen Ring oder in zwei miteinander verbundenen heterocyclischen Ringen, die entweder ein oder zwei Ringglieder gemeinsam haben oder durch ein Brückenglied miteinander verknüpft sind, wenigstens 2 Ringsegmente der Formel

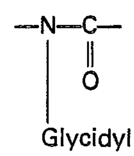

oder wenigstens ein Ringsegment der Formel

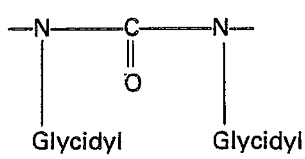

enthalten, in denen der Glycidylrest der Formel

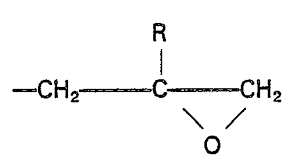

entspricht, in der R Wasserstoff oder der Methylrest ist, und daß sie weiterhin, sofern diese Ringsegmente nicht selbst zum Ring geschlossen sind, Kohlenstoff und gegebenenfalls zusätzlich Stickstoff als Ringschlußglieder enthalten, und daß sie als weitere Substituenten an den heterocyclischen Ringen Halogene oder unsubstituierte Kohlenwasserstoffreste mit nicht mehr als 8 C-Atomen aufweisen können, wobei als Heterocyclen Isocyanurate, Urazole und Hydantoine ausgenommen sind und wobei die darin

vorliegenden Polyglycidyl-substituierten N-Heterocyclen das Molekulargewicht von 1 000 nicht überschreiten.

2. Arzneimittelzubereitungen nach Anspruch 1, dadurch gekennzeichnet, daß die cytostatischen Wirkstoffe ausgewählt sind aus zwei- bis drei Glycidylgruppen aufweisenden heterocyclischen Verbindungen aus der Gruppe Ureide wie Uracile, Glykolurile oder Benzimidazolone; Polycarbonsäureimide und Barbitursäurederivate.

3. Arzneimittelzubereitungen nach Ansprüchen 1 bis 2, dadurch gekennzeichnet, daß die cytostatischen Wirkstoffe nur einen N-Glycidylrest in Imid- oder Amidform in einem Ring und wenigstens einen weiteren solchen N-Glycidylrest in einem weiteren heterocylischen Ring des gleichen Moleküls aufweisen, wobei diese Ringe verbunden oder miteinander verschmolzen sind.

4. Arzneimittelzubereitungen nach einem der Ansprüche 1 bis 3, dadurch gekennzeichnet, daß die N-Glycidylreste Bestandteil von heterocyclischen Ringen mit bis zu 10 Ringgliedern, vorzugsweise mit 4 bis 7 Ringgliedern und insbesondere 5 und/oder 6 Ringgliedern sind.

5. Arzneimittelzubereitungen nach einem der Ansprüche 1 bis 4, dadurch gekennzeichnet, daß sie solche mit mehreren Glycidylresten substituierte N-Heterocyclen enthalten, die als Substituenten an dem (den) heterocyclischen Ring(en) Kohlenwasserstoffreste aufweisen, die jeweils bis zu 8 C-Atome besitzen.

6. Arzneimittelzubereitungen nach einem der Ansprüche 1 bis 5, enthaltend Verbindungen der allgemeinen Formel II

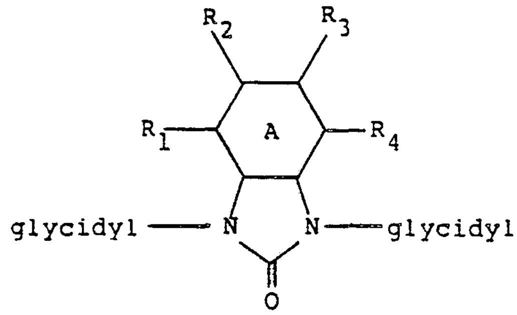

(II)

in der Glycidyl die angegebene Bedeutung hat, A ein aromatisches, cycloaliphatisches oder olefinisch ungesättigtes 6-Ring-System ist, welches auch ein Heteroatom enthalten kann, und die Reste $R_1$ bis $R_4$ die folgende Bedeutung haben: gleiche oder verschiedene Reste aus der Gruppe Wasserstoff, Halogen, Kohlenwasserstoffreste mit jeweils nicht mehr als 8 C-Atomen.

7. Arzneimittelzubereitungen nach einem der Ansprüche 1 bis 5, enthaltend Verbindungen der allgemeinen Formel III

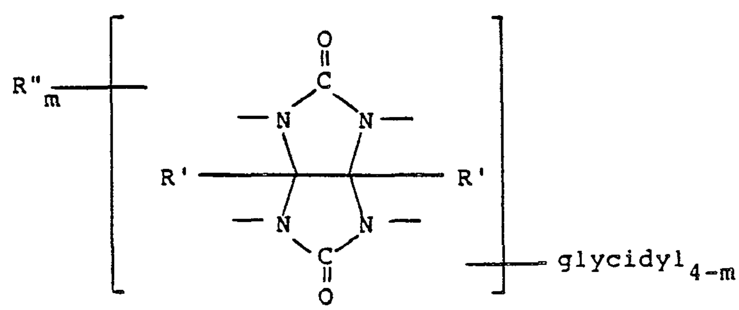

(III)

in der Glycidyl die angegebene Bedeutung hat und die Reste R′, R″ und m die folgen

R′: gleiche oder verschiedene Reste aus der Gruppe Wasserstoff, Kohlenwasserstoffreste mit jeweils bis zu 8 C-Atomen,

R″: gleiche oder verschiedene Reste aus der Gruppe Wasserstoff, Kohlenwasserstoffreste mit jeweils bis zu 4 C-Atomen,

m: 0, 1 oder 2.

8. Arzneimittelzubereitungen nach einem der Ansprüche 1 bis 5, enthaltend Verbindungen der allgemeinen Formel IV

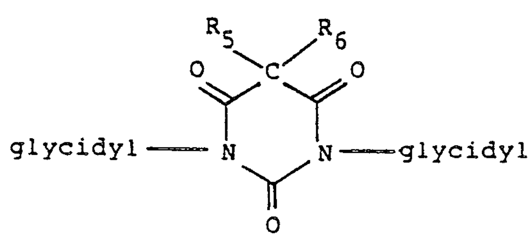

(IV)

in der die Reste $R_5$ und $R_6$ die folgende Bedeutung haben:
$R_5$ und $R_6$:

(1) gleiche oder verschiedene Reste aus der Gruppe Wasserstoff und Kohlenwasserstoffreste mit jeweils bis zu 12 C-Atomen.

(2) $R_5$ und $R_6$ sind gemeinsam zu einem Ringsystem geschlossen.

9. Arzneimittelzubereitungen nach einem der Ansprüche 1 bis 5, enthaltend Verbindungen der allgemeinen Formel V

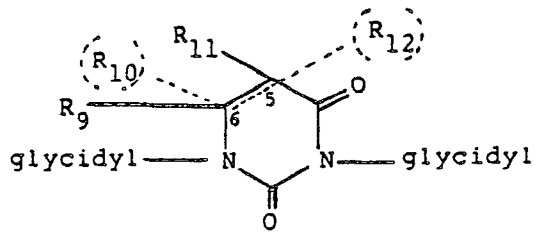

(V)

in der die Reste $R_9$, $R_{10}$ $R_{11}$ und $R_{12}$ die folgende Bedeutung haben:
$R_9$ bis $R_{12}$:

(1) gleiche oder verschiedene Reste aus der Gruppe Wasserstoff, Halogen und Kohlenwasserstoffreste mit jeweils bis zu 8 C-Atomen.

(2) wenigstens zwei der Reste $R_9$ bis $R_{12}$ sind gemeinsam zu einem Ringsystem geschlossen.

(3) wenigstens eins der Paare $R_9$/$R_{10}$ beziehungsweise $R_{11}$/$R_{12}$ bilden zusammen den Rest

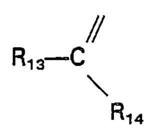

worin $R_{13}$ und $R_{14}$ die Bedeutung von $R_9$ bis $R_{12}$ zu (1) oder (2) haben, während Glycidyl die angegebene Bedeutung hat.

10. Arzneimittelzubereitung nach einem der Ansprüche 1 bis 5 dadurch gekennzeichnet, daß Polycarbonsäureimide mit wenigstens 2 mit Glycidylresten substituierten cyclischen Imidgruppen enthalten sind, ausgewählt aus der Gruppe:
N,N'-Diglycidyl-pyromellithsäure-diimid (1,2,4,5-Benzoltetracarbonsäurediimid)
N,N'-Diglycidyl-1,2,3,4-benzoltetracarbonsäurediimid
N,N'-Diglycidyl-1,4,5,8-naphthalintetracarbonsäurediimid
N,N'-Diglycidyl-2,3,4,5-pyridintetracarbonsäurediimid
N,N'-Diglycidyl-2,3,5,6-pyridintetracarbonsäurediimid
N,N'-Diglycidyl-1,2,4,5-Cyclohexantetracarbonsäurediimid
N,N'-Diglycidyl-1,1,2,2-ethantetracarbonsäurediimid
N,N'-Diglycidyl-1,2,3,4-butantetracarbonsäuretriimid
N,N',N''-Triglycidyl-benzolhexacarbonsäuretriimid
N,N'-Diglycidyl-tetrahydrofurantetracarbonsäurediimid

11. Arzneimittel nach einem der Ansprüche 1 bis 10, dadurch gekennzeichnet, daß beim Vorliegen von Kohlenwasserstoffresten als Substituenten an dem beziehungsweise den heterocyclischen Ringen diese Kohlenwasserstoffreste zusammen nicht mehr als 12 C-Atome, insbesondere nicht mehr als 8 C-Atome aufweisen, wobei solche Reste mit bis zu 4 C-Atomen besonders bevorzugt sein können.

12. Arzneimittel nach einem der Ansprüche 1 bis 11, dadurch gekennzeichnet, daß die an dem heterocyclischen Ringsystem vorliegenden Substituenten insgesamt nicht mehr als 12 C-Atome, insbesondere nicht mehr als 8 C-Atome aufweisen, wobei es besonders bevorzugt sein kann, daß diese Reste zusammen nicht mehr als 4 C-Atome besitzen.

13. Arzneimittel nach einem der Ansprüche 1 bis 12, dadurch gekennzeichnet, daß sie Polyglycidylverbindungen mit 2 bis 4, insbesondere mit 2 oder 3 Glycidylresten an N-Atomen in Amid- und/oder in Imidbindung enthalten.

14. Arzneimittel nach einem der Ansprüche 1 bis 13, dadurch gekennzeichnet, daß sie die Polyglycidylsubstituierten N-Heterocyclen in Abmischung mit üblichen pharmakologischen Hilfs- und/oder Trägerstoffen enthalten.

**Patentansprüche für den Vertragsstaat: AT**

1. Verfahren nach Herstellung von Arzneimittelzubereitungen mit cytostatischer Wirksamkeit auf Basis von Polyglycidyl-substituierten N-heterocyclischen organischen Ringverbindungen, dadurch gekennzeichnet, daß die Polyglycidyl-substituierten N-heterocyclischen organischen Ringverbindungen in einem heterocyclischen Ring oder in zwei miteinander verbundenen heterocyclischen Ringen, die

entweider ein oder zwei Ringglieder gemeinsam haben oder durch ein Brückenglied miteinander verknüpft sind, wenigstens 2 Ringsegmente der Formel

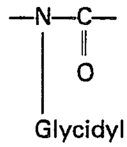

oder wenigstens ein Ringsegment der Formel

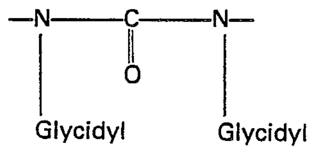

enthalten, in denen der Glycidylrest der Formel

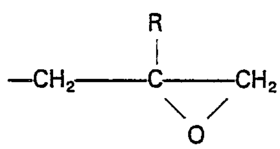

entspricht, in der R Wasserstoff oder der Methylrest ist, und daß sie weiterhin, sofern diese Ringsegmente nicht selbst zum Ring geschlossen sind, Kohlenstoff und gegebenenfalls zusätzlich Stickstoff als Ringschlußglieder enthalten, und daß sie als weitere Substituenten an den heterocyclischen Ringen Halogene oder unsubstituierte Kohlenwasserstoffreste mit nicht mehr als 8 C-Atomen aufweisen können, wobei als Heterocyclen Isocyanurate, Urazole und Hydantoine ausgenommen sind und wobei die darin vorliegenden Polyglycidyl-substituierten N-Heterocyclen das Molekulargewicht von 1 000 nicht überschreiten.

2. Verfahren zur Herstellung von Arzneimittelzubereitungen nach Anspruch 1, dadurch gekennzeichnet, daß die cytostatischen Wirkstoffe ausgewählt sind aus zwei- bis drei Glycidylgruppen aufweisenden heterocyclischen Verbindungen aus der Gruppe Ureide wie Uracile, Glykolurile oder Benzimidazolone; Polycarbonsäureimide und Barbitursäurederivate.

3. Verfahren zur Herstellung von Arzneimittelzubereitungen nach Ansprüchen 1 bis 2, dadurch gekennzeichnet, daß die cytostatischen Wirkstoffe nur einen N-Glycidylrest in Imid- oder Amidform in einem Ring und wenigstens einen weiteren solchen N-Glycidylrest in einem weiteren heterocylischen Ring des gleichen Moleküls aufweisen, wobei diese Ringe verbunden oder miteinander verschmolzen sind.

4. Verfahren zur Herstellung von Arzneimittelzubereitungen nach einem der Ansprüche 1 bis 3, dadurch gekennzeichnet, daß die N-Glycidylreste Bestandteil von heterocyclischen Ringen mit bis zu 10 Ringgliedern, vorzugsweise mit 4 bis 7 Ringgliedern und insbesondere 5 und/oder 6 Ringgliedern sind.

5. Verfahren zur Herstellung von Arzneimittelzubereitungen nach einem der Ansprüche 1 bis 4, dadurch gekennzeichnet, daß sie solche mit mehreren Glycidylresten substituierte N-Heterocyclen enthalten, die als Substituenten an dem (den) heterocyclischen Ring(en) Kohlenwasserstoffreste aufweisen, die jeweils bis zu 8 C-Atome besitzen.

6. Verfahren zur Herstellung von Arzneimittelzubereitungen nach einem der Ansprüche 1 bis 5, enthaltend Verbindungen der allgemeinen Formel II

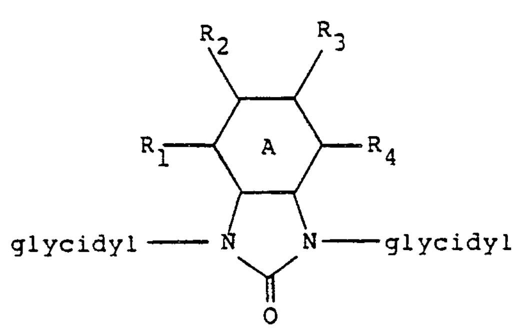

(II)

in der Glycidyl die angegebene Bedeutung hat, A ein aromatisches, cycloaliphatisches oder olefinisch ungesättigtes 6-Ring-System ist, welches auch ein Heteroatom enthalten kann, und die Reste $R_1$ bis $R_4$ die

folgende Bedeutung haben: gleiche oder verschiedene Reste aus der Gruppe Wasserstoff, Halogen, Kohlenwasserstoffreste mit jeweils nicht mehr als 8 C-Atomen.

7. Verfahren zur Herstellung von Arzneimittelzubereitungen nach einem der Ansprüche 1 bis 5, enthaltend Verbindungen der allgemeinen Formel III

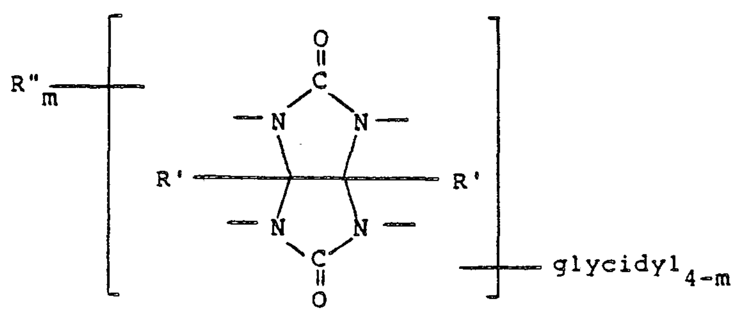

(III)

in der Glycidyl die angegebene Bedeutung hat und die Reste R', R'' und m die folgen

R': gleiche oder verschiedene Reste aus der Gruppe Wasserstoff, Kohlenwasserstoffreste mit jeweils bis zu 8 C-Atomen

R'': gleiche oder verschiedene Reste aus der Gruppe Wasserstoff, Kohlenwasserstoffreste mit jeweils bis zu 4 C-Atomen,

m: 0, 1 oder 2.

8. Verfahren zur Herstellung von Arzneimittelzubereitungen nach einem der Ansprüche 1 bis 5, enthaltend Verbindungen der allgemeinen Formel IV

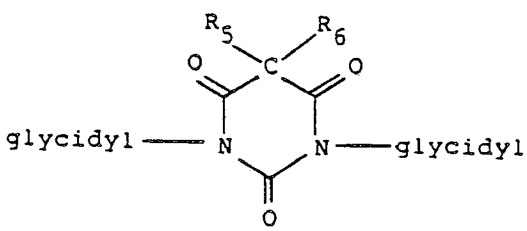

(IV)

in der die Reste $R_5$ und $R_6$ die folgende Bedeutung haben:

$R_5$ und $R_6$:

(1) gleiche oder verschiedene Reste aus der Gruppe Wasserstoff und Kohlenwasserstoffreste mit jeweils bis zu 12 C-Atomen.

(2) $R_5$ und $R_6$ sind gemeinsam zu einem Ringsystem geschlossen.

9. Verfahren zur Herstellung von Arzneimittelzubereitungen nach einem der Ansprüche 1 bis 5, enthaltend Verbindungen der allgemeinen Formel V

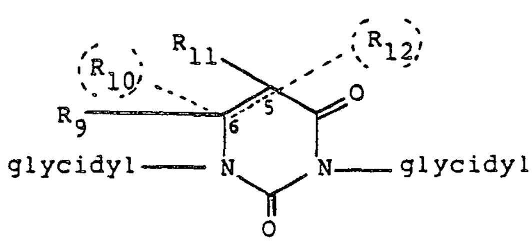

(V)

in der die Reste $R_9$, $R_{10}$ $R_{11}$ und $R_{12}$ die folgende Bedeutung haben:

$R_9$ bis $R_{12}$:

(1) gleiche oder verschiedene Reste aus der Gruppe Wasserstoff, Halogen und Kohlenwasserstoffreste mit jeweils biz zu 8 C-Atomen, die auch substituiert sein können

(2) wenigstens zwei der Reste $R_9$ bis $R_{12}$ sind gemeinsam zu einem Ringsystem geschlossen

(3) wenigstens eins der Paare $R_9$/$R_{10}$ bzw. $R_{11}$/$R_{12}$ bilden zusammen den Rest

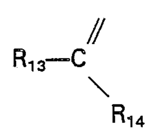

worin $R_{13}$ und $R_{14}$ die Bedeutung von $R_9$ bis $R_{12}$ zu (1) oder (2) haben, während Glycidyl die angegebene Bedeutung hat.

10. Verfahren zur Herstellung von Arzneimittelzubereitung nach einem der Ansprüche 1 bis 5, dadurch gekennzeichnet, daß Polycarbonsäureimide mit wenigstens 2 mit Glycidylresten substituierten cylischen Imidgruppen enthalten sind, ausgewählt aus der Gruppe:

N,N'-Diglycidyl-pyromellithsäure-diimid (1,2,4,5-Benzol-tetracarbonsäurediimid)
N,N'-Diglycidyl-1,2,3,4-benzoltetracarbonsäurediimid
N,N'-Diglycidyl-1,4,5,8-naphthalintetracarbonsäurediimid
N,N'-Diglycidyl-2,3,4,5-pyridintetracarbonsäurediimid
N,N'-Diglycidyl-2,3,5,6-pyridintetracarbonsäurediimid
N,N'-Diglycidyl-1,2,4,5-Cyclohexantetracarbonsäurediimid
N,N'-Diglycidyl-1,1,2,2-ethantetracarbonsäurediimid
N,N'-Diglycidyl-1,2,3,4-butantetracarbonsäuretriimid
N,N',N''-Triglycidyl-benzolhexacarbonsäuretriimid
N,N'-Diglycidyl-tetrahydrofurantetracarbonsäurediimid

11. Verfahren zur Herstellung von Arzneimittelzubereitung nach einem der Ansprüche 1 bis 10, dadurch gekennzeichnet, daß beim Vorliegen von Kohlenwasserstoffresten als Substituenten den dem beziehungsweise den heterocyclischen Ringen diese Kohlenwasserstoffreste zusammen nicht mehr als 12 C-Atome, insbesondere nicht mehr als 8 C-Atome aufweisen, wobei solche Reste mit bis 4 C-Atomen besonders bevorzugt sein können.

12. Verfahren zur Herstellung von Arzneimittelzubereitungen nach einem der Ansprüche 1 bis 11, dadurch gekennzeichnet, daß die an dem heterocyclischen Ringsystem vorliegenden Substituenten insgesamt nicht mehr als 12 C-Atome, insbesondere nicht mehr als 8 C-Atome aufweisen, wobei es besonders bevorzugt sein kann, daß diese Reste zusammen nicht mehr als 4 C-Atome besitzen.

13. Verfahren zur Herstellung von Arzneimittelzubereitungen nach einem der Ansprüche 1 bis 12, dadurch gekennzeichnet, daß sie Polyglycidylverbindungen mit 2 bis 4, insbesondere mit 2 oder 3 Glycidylresten an N-Atomen in Amid- und/oder in Imidbindung enthalten.

14. Verfahren zur Herstellung von Arzneimittelzubereitungen nach einem der Ansprüche 1 bis 13, dadurch gekennzeichnet, daß sie die Polyglycidylsubstituierten N-Heterocyclen in Abmischung mit üblichen pharmakologischen Hilfs- und/oder Trägerstoffen enthalten.

**Claims for the Contracting States: BE CH DE FR GB IT LI NL SE**

1. Cytostatic medicinal preparations based on polyglycidyl-substituted N-heterocyclic organic ring compounds, characterized in that they contain in one heterocyclic ring or in two interconnected heterocyclic rings, which either have one or two ring members in common or are attached to one another by a bridge member, at least two ring segments corresponding to the following formula

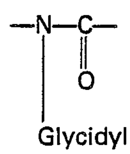

or at least one ring segment corresponding to the following formula

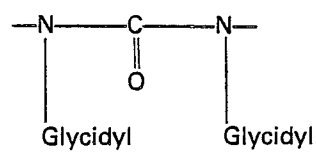

in which the glycidyl residue corresponds to the following formula

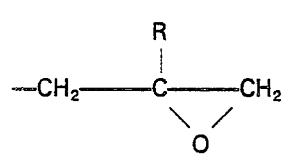

in which R is hydrogen or the methyl group, and in that, providing these ring segments are not themselves closed to form the ring, they further contain carbon and, optionally, nitrogen as ring-closing elements and in that they may contain halogens or unsubstituted hydrocarbon radicals containing no more than 8 C-atoms as further substituents on the heterocyclic rings, isocyanurates, urazoles and hydantoins being

excluded as heterocycles and the polyglycidyl-substituted N-heterocycles present therein not exceeding a molecular weight of 1000.

2. Medicinal preparations as claimed in Claim 1, characterized in that the cytostatic agents are selected from heterocyclic compounds containing two to three glycidyl groups from the group comprising ureides, such as uracils, glycolurils or benzimidazolones; polycarboxylic acid imides and barbituric acid derivatives.

3. Medicinal preparations as claimed in Claims 1 and 2, characterized in that the cytostatic agents contain only one N-glycidyl residue in imide or amide form in one ring and at least one other such N-glycidyl residue in another heterocyclic ring of the same molecule, these rings being attached to or fused with one another.

4. Medicinal preparations as claimed in any of Claims 1 to 3, characterized in that the N-glycidyl residues are part of heterocyclic rings containing up to 10 ring members, preferably from 4 to 7 ring members and more especially 5 and/or 6 ring members.

5. Medicinal preparations as claimed in any of Claims 1 to 4, characterized in that they contain N-heterocycles substituted by several glycidyl residues of the type which contain as substituents on the heterocyclic ring(s) hydrocarbon radicals containing up to 8 C-atoms.

6. Medicinal preparations as claimed in any of Claims 1 to 5 containing compounds corresponding to the following general formula

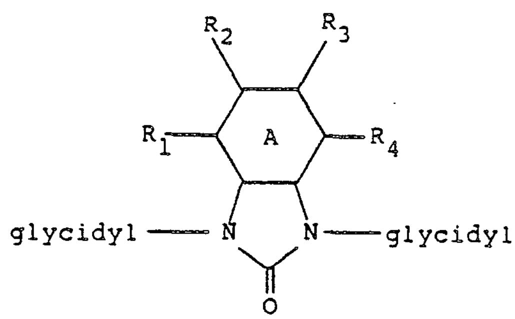

(II)

in which glydicyl is as defined above, A is an aromatic, cycloaliphatic or olefinically unsaturated 6-ring system which may also contain a hetero atom and the radicals $R_1$ to $R_4$ have the following meaning: they may be the same or different and represent hydrogen, halogen, hydrocarbon radicals containing no more than 8 carbon atoms.

7. Medicinal preparations as claimed in any of Claims 1 to 5 containing compounds corresponding to the following general formula

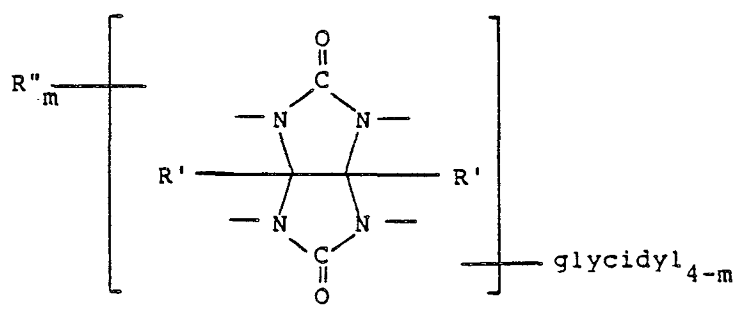

(III)

in which glycidyl is as defined above and the radicals R′, R″ and m have the following meanings:

R′: may be the same or different and represent hydrogen or hydrocarbon radicals containing up to 8 carbon atoms,

R″: may be the same or different and represents hydrogen or hydrocarbon radicals containing up to 4 carbon atoms,

m: 0,. 1 or 2.

8. Medicinal preparations as claimed in any of Claims 1 to 5 containing compounds corresponding to the following general formula

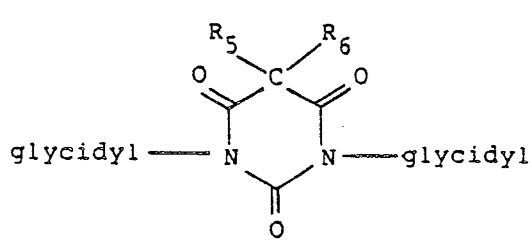

(IV)

in which the radicals $R_5$ and $R_6$ have the following meanings:

$R_5$ and $R_6$:

(1) may be the same or different and represent hydrogen or hydrocarbon radicals containing up to 12 C-atoms,

(2) $R_5$ and $R_6$ are closed together to form a ring system.

9. Medicinal preparations as claimed in any of Claims 1 to 5 containing compounds corresponding to the following general formula

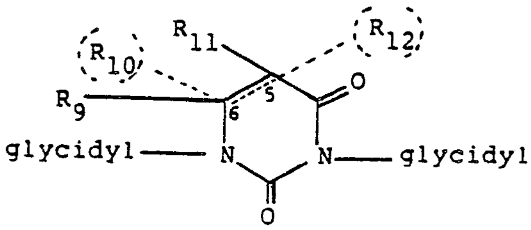

(V)

in which the radicals $R_9$, $R_{10}$, $R_{11}$ and $R_{12}$ have the following meanings:

$R_9$—$R_{12}$:

(1) may be the same or different and represent hydrogen, halogen or hydrocarbon radicals containing up to 8 C-atoms,

(2) at least two of the radicals $R_9$ to $R_{12}$ are closed together to form a ring system,

(3) at least one of the pairs $R_9/R_{10}$ and $R_{11}/R_{12}$ together form the radical

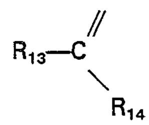

where $R_{13}$ and $R_{14}$ have the same meanings as $R_9$ to $R_{12}$ as defined in (1) or (2) while glycidyl is as defined above.

10. A medicinal preparation as claimed in any of Claims 1 to 5, characterized in that it contains polycarboxylic acid imides containing at least two glycidyl-substituted cyclic imide groups selected from the group:

N,N'-diglycidyl-pyromellitic acid diimide (1,2,4,5-benzene tetracarboxylic acid diimide)
N,N'-diglycidyl-1,2,3,4-benzene tetracarboxylic acid diimide
N,N'-diglycidyl-1,4,5,8-naphthalene tetracarboxylic acid diimide
N,N'-diglycidyl-2,3,4,5-pyridine tetracarboxylic acid diimide
N,N'-diglycidyl-2,3,5,6-pyridine tetracarboxylic acid diimide
N,N'-diglycidyl-1,2,4,5-cyclohexane tetracarboxylic acid diimide
N,N'-diglycidyl-1,1,2,2-ethane tetracarboxylic acid diimide
N,N'-diglycidyl-1,2,3,4-butane tetracarboxylic acid triimide
N,N', N''-triglycidyl benzene hexacarboxylic acid triimide
N,N'-diglycidyl-tetrahydrofuran tetracarboxylic acid diimide.

11. Medicinal preparations as claimed in any of Claims 1 to 10, characterized in that, where hydrocarbon radicals are present as substituents on the heterocyclic ring(s), these hydrocarbon radicals together contain no more than 12 and preferably no more than 8 C-atoms; hydrocarbon radicals containing up to 4 C-atoms may be particularly preferred.

12. Medicinal preparations as claimed in any of Claims 1 to 11, characterized in that the substituents on the heterocyclic ring system contain in all no more than 12 C-atoms and preferably no more than 8 C-atoms; it may be particularly preferred for these radicals together to contain no more than 4 C-atoms.

13. Medicinal preparations as claimed in any of Claims 1 to 12, characterized in that they contain polyglycidyl compounds containing from 2 to 4 and more especially 2 or 3 glycidyl residues at N-atoms in amide and/or in imide form.

14. Medicinal preparations as claimed in any of Claims 1 to 13, characterized in that they contain the polyglycidyl-substituted N-heterocycles in admixture with standard pharmacological auxiliaries and/or carriers.

**Claims for the Contracting State: AT**

1. A process for the production of cytostatic medicinal preparations based on polyglicidyl-substituted N-heterocyclic organic ring compounds, characterized in that the polyglycidyl-substituted N-heterocyclic organic ring compounds contain in one heterocyclic ring or in two interconnected heterocyclic rings, which either have one or two ring members in common or are attached to one another by a bridge member, at least two ring segments corresponding to the following formula

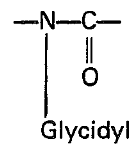

or at least one ring segment corresponding to the following formula

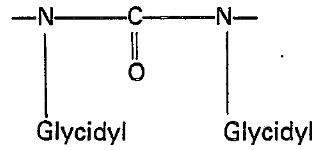

in which the glycidyl residue corresponds to the following formula

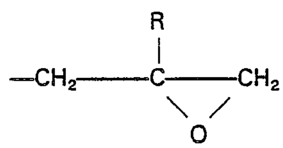

in which R is hydrogen or the methyl group, and in that, providing these ring segments are not themselves closed to form the ring, they further contain carbon and, optionally, nitrogen as ring-closing elements and in that they may contain halogens or unsubstituted hydrocarbon radicals containing no more than 8 C-atoms as further substituents on the heterocyclic rings, isocyanurates, urazoles and hydantoins being excluded as heterocycles and the polyglycidyl-substituted N-heterocycles present therein not exceeding a molecular weight of 1000.

2. A process for the production of medicinal preparations as claimed in Claim 1, characterized in that the cytostatic agents are selected from heterocyclic compounds containing two to three glycidyl groups from the group comprising ureides, such as uracils, glycolurils or benzimidazolones; polycarboxylic acid imides and barbituric acid derivatives.

3. A process for the production of medicinal preparations as claimed in Claims 1 and 2, characterized in that the cytostatic agents contain only one N-glycidyl residue in imide or amide form in one ring and at least one other such N-glycidyl residue in another heterocyclic ring of the same molecule, these rings being attached to or fused with one another.

4. A process for the production of medicinal preparations as claimed in any of Claims 1 to 3, characterized in that the N-glycidyl residues are part of heterocyclic rings containing up to 10 ring members, preferably from 4 to 7 ring members and more especially 5 and/or 6 ring members.

5. A process for the production of medicinal preparations as claimed in any of Claims 1 to 4, characterized in that they contain N-heterocycles substituted by several glycidyl residues of the type which contain as substituents on the heterocyclic ring(s) hydrocarbon radicals containing up to 8 C-atoms.

6. A process for the production of medicinal preparations as claimed in any of Claims 1 to 5 containing compounds corresponding to the following general formula

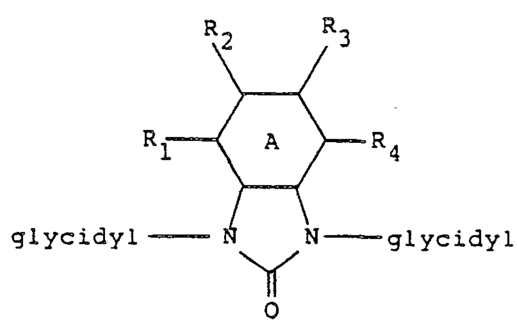

(II)

in which glydicyl is as defined above, A is an aromatic, cycloaliphatic or olefinically unsaturated 6-ring system which may also contain a hetero atom and the radicals $R_1$ to $R_4$ have the following meaning: they may be the same or different and represent hydrogen, halogen, hydrocarbon radicals containing no more than 8 carbon atoms.

7. A process for the production of medicinal preparations as claimed in any of Claims 1 to 5 containing compounds corresponding to the following general formula

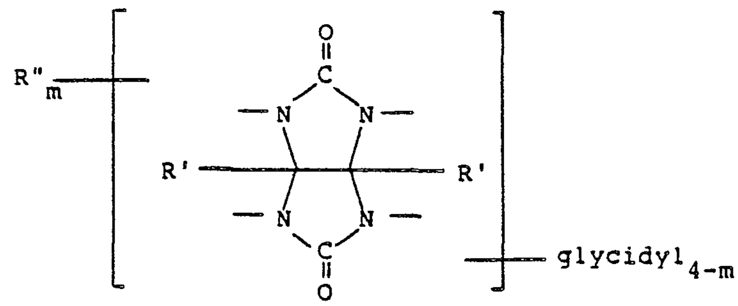

(III)

in which glycidyl is as defined above and the radicals R′, R″ and m have the following meanings:

R′: may be the same or different and represent hydrogen or hydrocarbon radicals containing up to 8 carbon atoms,

R″: may be the same or different and represents hydrogen or hydrocarbon radicals containing up to 4 carbon atoms,

m: 0,. 1 or 2.

8. A process for the production of medicinal preparations as claimed in any of Claims 1 to 5 containing compounds corresponding to the following general formula

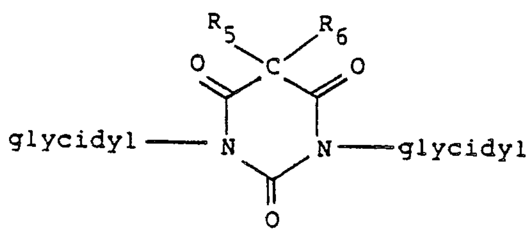

(IV)

in which the radicals $R_5$ and $R_6$ have the following meanings:

$R_5$ and $R_6$:

(1) may be the same or different and represent hydrogen or hydrocarbon radicals containing up to 12 C-atoms,

(2) $R_5$ and $R_6$ are closed together to form a ring system.

9. A process for the production of medicinal preparations as claimed in any of Claims 1 to 5 containing compounds corresponding to the following general formula

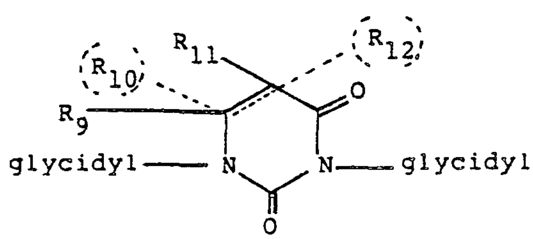

(V)

in which the radicals $R_9$, $R_{10}$, $R_{11}$ and $R_{12}$ have the following meanings:

$R_9$—$R_{12}$:

(1) may be the same or different and represent hydrogen, halogen or hydrocarbon radicals containing up to 8 C-atoms, which can also be substituted,

(2) at least two of the radicals $R_9$ to $R_{12}$ are closed together to form a ring system,

(3) at least one of the pairs $R_9/R_{10}$ and $R_{11}/R_{12}$ together form the radical

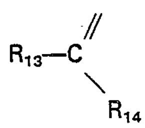

where $R_{13}$ and $R_{14}$ have the same meanings as $R_9$ to $R_{12}$ as defined in (1) or (2) while glycidyl is as defined above.

10. A process for the production of medicinal preparations as claimed in any of Claims 1 to 5, characterized in that they contain polycarboxylic acid imides containing at least two glycidyl-substituted cyclic imide groups selected from the group:

N,N'-diglycidyl-pyromellitic acid diimide (1,2,4,5-benzene tetracarboxylic acid diimide)
N,N'-diglycidyl-1,2,3,4-benzene tetracarboxylic acid diimide
N,N'-diglycidyl-1,4,5,8-naphthalene tetracarboxylic acid diimide
N,N'-diglycidyl-2,3,4,5-pyridine tetracarboxylic acid diimide
N,N'-diglycidyl-2,3,5,6-pyridine tetracarboxylic acid diimide
N,N'-diglycidyl-1,2,4,5-cyclohexane tetracarboxylic acid diimide
N,N'-diglycidyl-1,1,2,2-ethane tetracarboxylic acid diimide
N,N'-diglycidyl-1,2,3,4-butane tetracarboxylic acid triimide
N,N', N''-triglycidyl benzene hexacarboxylic acid triimide
N,N'-diglycidyl-tetrahydrofuran tetracarboxylic acid diimide.

11. A process for the production of medicinal preparations as claimed in any of Claims 1 to 10, characterized in that, where hydrocarbon radicals are present as substituents on the heterocyclic ring(s), these hydrocarbon radicals together contain no more than 12 and preferably no more than 8 C-atoms; hydrocarbon radicals containing up to 4 C-atoms may be particularly preferred.

12. A process for the production of medicinal preparations as claimed in any of Claims 1 to 11, characterized in that the substituents on the heterocyclic ring system contain in all no more than 12 C-atoms and preferably no more than 8 C-atoms; it may be particularly preferred for these radicals together to contain no more than 4 C-atoms.

13. A process for the production of medicinal preparations as claimed in any of Claims 1 to 12, characterized in that they contain polyglycidyl compounds containing from 2 to 4 and more especially 2 or 3 glycidyl residues at N-atoms in amide and/or in imide form.

14. A process for the production of medicinal preparations as claimed in any of Claims 1 to 13, characterized in that they contain the polyglycidyl-substituted N-heterocycles in admixture with standard pharmacological auxiliaries and/or carriers.

**Revendications pour les Etats contractants: BE CH DE FR GB IT LI NL SE**

1. Préparation médicamenteuses à activité cytostatique, à base de composés cycliques organiques N-hétérocycliques avec substituants polyglycidyle, caractérisés en ce que dans un noyau hétérocyclique ou dans deux noyaux hétérocycliques reliés entre eux, qui ou bien ont un ou deux chaînons en commun ou sont reliés entre eux par un chaînon de pontage, ils contiennent au moins 2 segments nucléaires de formule:

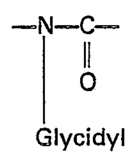

ou au moins un segment de formule:

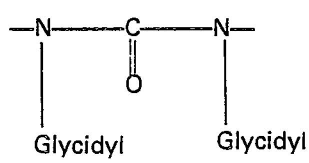

dans lesquels le radical glycidyle correspond à la formule

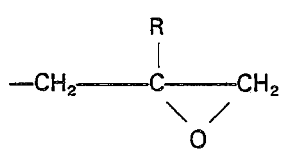

dans laquelle R est de l'hydrogène ou le radical méthyle et en ce qu'en outre, pour autant que ces segments nucléaires ne sont pas déjà eux-mêmes fermés en un cycle, ils contiennent du carbone et éventuellement en plus d l'azote comme chaînons de fermeture du cycle, et en ce que comme autres substituants sur les noyaux hétérocycliques ils peuvent contenir des halogènes ou des radicaux hydrocarbonés non substitués n'ayant pas plus de 8 atomes de carbone, en l'occurrence en tant que hétérocycles sont exceptés les isocyanurates, les urazols et les hydantoïnes et en l'occurrence les N-hétérocycles avec substituants polyglycidyle qui y sont présents ne dépassant pas le poids moléculaire de 1000.

2. Préparations médicamenteuses selon la revendication 1, caractérisées en ce que les matières actives cytostatiques sont sélectionnées à partir de composés hétérocycliques présentant deux à trois groupes

glycidyle du groupe des uréides, comme des uraciles, des glycolurils ou des benzimidazolones, des polycarboximides et des dérivés d'acide barbiturique.

3. Préparations médicamenteuses selon revendications 1 à 2, caractérisées en ce que les matières actives cytostatiques ne présentent qu'un radical N-glycidyle sous la forme imide ou amide dans un noyau et présentent au moins un autre radical N-glycidyle dans un autre noyau hétérocyclique de la même molécule, ces noyaux étant reliés ou fusionnés entre eux.

4. Préparations médicamenteuses selon l'une des revendications 1 à 3, caractérisées en ce que les radicaux N-glycidyle sont un constituant de noyaux hétérocycliques ayant jusqu'à 10 chaînons nucléaires, de préférence avec 4 à 7 chaînons nucléaires et en particulier 5 et/ou 6 chaînons nucléaires.

5. Préparations médicamenteuses selon l'un des revendications 1 à 4, caractérisées en ce qu'elles présentent des N-hétérocycles substitués par plusieurs radicaux glycidyle qui possèdent comme substituants sur le (ou les) noyau(x) hétérocyclique(s) des radicaux hydrocarbonés qui possèdent chacun jusqu'à 8 atomes de carbone.

6. Préparations médicamenteuses selon l'une des revendications 1 à 5, contenant des composés de formule générale II:

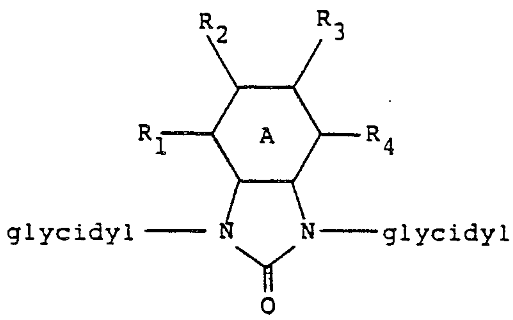

(II)

dans laquelle glycidyle a la signification indiquée, A est un système avec noyau en 6 aromatique, cycloaliphatique ou oléfiniquement insaturé qui peut aussi contenir un hétéroatome, les radicaux $R_1$ à $R_4$ ayant la signification suivante: des radicaux identiques ou différents appartenant au groupe hydrogène, halogène, radicaux hydrocarbonés ayant chacun au plus 8 atomes de carbone.

7. Préparations médicamenteuses selon l'une des revendications 1 à 5, contenant des composés de formule générale III:

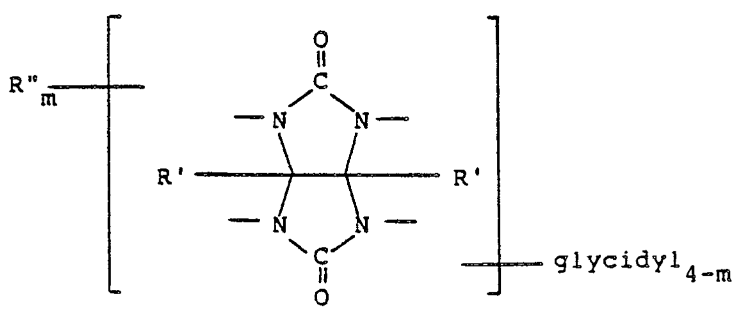

(III)

dans laquelle glycidyle a la signification indiquée et les radicaux R', R'' et m ont les significations suivantes:

R': des radicaux identiques ou différents du groupe hydrogène, radicaux hydrocarbonés ayant chacun jusqu'à 8 atomes de carbone,

R'': radicaux identiques ou différents du groupe hydrogène, radicaux hydrocarbonés ayant chacun jusqu'à 4 atomes de carbone,

m: 0, 1 ou 2.

8. Préparations médicamenteuses selon les revendications 1 à 5, contenant des composés de formule générale IV:

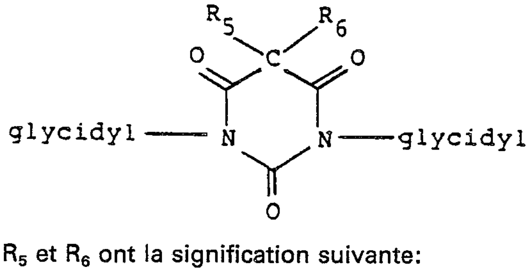

(IV)

dans laquelle les radicaux $R_5$ et $R_6$ ont la signification suivante:

$R_5$ et $R_6$:

(1) des radicaux identiques ou différents du groupe hydrogène et radicaux hydrocarbonés ayant chacun jusqu'à 12 atomes de carbone.

(2) $R_5$ et $R_6$ sont fermés entre eux en un système nucléaire.

9. Préparations médicamenteuses selon l'un des revendications 1 à 5, contenant des composés de formule générale V:

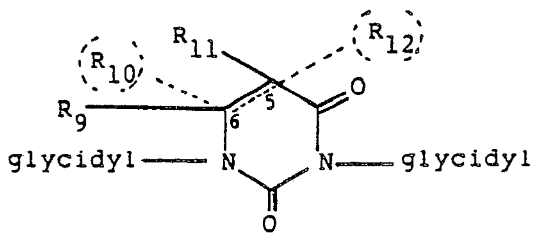

(V)

dans laquelle les radicaux $R_9$, $R_{10}$, $R_{11}$ et $R_{12}$ ont la signification suivante:

$R_9$ à $R_{12}$:

(1) des radicaux identiques ou différents du groupe hydrogène, halogène et radicaux hydrocarbonés ayant chacun jusqu'à 8 atomes de carbone,

(2) au moins deux des radicaux $R_9$ à $R_{12}$ sont fermés entre eux en un système nucléaire

(3) au moins une des paires $R_9/R_{10}$ ou $R_{11}/R_{12}$ forment ensemble le radical

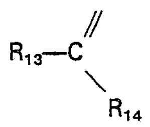

où $R_{13}$ et $R_{14}$ ont la signification de $R_9$ à $R_{12}$ en (1) ou en (2), tandis que glycidyle a la signification indiquée.

10. Préparation médicamenteuse selon l'une des revendications 1 à 5, caractérisée en ce qu'y sont contenues des polycarboximides avec au moins 2 groupes imide cycliques substitués par des radicaux glycidyle, choisies dans le groupe:

diimide d'acide N,N'-diglycidyl-pyromellitique (diimide d'acide 1,2,4,5-benzène-tétracarboxylique),
diimide d'acide N,N'-diglycidyl-1,2,3,4,-benzène-tétracarboxylique,
diimide d'acide N,N'-diglycidyl-1,4,5,8-naphtalène-tétracarboxylique
diimide d'acide N,N'-diglycidyl-2,3,4,5-pyridine-tétracarboxylique
diimide d'acide N,N'-diglycidyl-2,3,5,6-pyridine-tétracarboxylique
diimide d'acide N,N'-diglycidyl-1,2,4,5-cyclohexane-tétracarboxylique
diimide d'acide N,N'-diglycidyl-1,1,2,2-éthane-tétracarboxylique
triimide d'acide N,N'-diglycidyl-1,2,3,4-butane-tétracarboxylique
triimide d'acide N,N',N''-triglycidyl-benzène-hexacarboxylique
diimide d'acide N,N'-diglycidyl-tétrahydrofuranne-tétracarboxylique.

11. Médicaments selon l'une des revendications 1 à 10, caractérisés en ce qu'en cas de présence de radicaux hydrocarbonés comme substituants sur le ou les noyaux hétérocycliques, ces radicaux hydrocarbonés ensemble ne présentent pas plus de 12 atomes de carbone, en particulier pas plus de 8 atomes de carbone, pouvant être particulièrement préférés les radicaux ayant jusqu'à 4 atomes de carbone.

12. Médicaments selon l'une des revendications 1 à 11, caractérisés en ce que les substituants pésents sur le système nucléaire hétérocylique en tout ne possèdent pas plus de 12 atomes de carbone, en particulier pas plus de 8 atomes de carbone, pouvant être particulièrement préféré le fait que ces radicaux dans leur ensemble ne possèdent pas plus de 4 atomes de carbone.

13. Médicaments selon l'une des revendications 1 à 12, caractérisés en ce qu'ils contiennent des composés polyglycidyliques ayant 2 à 4, en particulier 2 ou 3 radicaux glycidyle sur les atomes N dans la liaison amide et/ou imide.

14. Médicaments selon l'une des revendications 1 à 13, caractérisés en ce qu'ils contiennent les N-hétérocycles polyglycidyl-substitués en mélange avec les matières auxiliaires et/ou de support en usage pharmacologique.

**Revendications pour l'Etat contractant: AT**

1. Procédé de fabrication de préparations médicamenteuses à activité cytostatique, à base de composés cycliques organiques N-hétérocycliques polyglycidyl-substitués, caractérisé en ce que les composés cycliques organiques N-hétérocycliques polyglycidyl-substitués contiennent dans un noyau hétérocyclique ou dans deux noyaux hétérocycliques reliés entre eux qui ont soit en commun un ou deux chaînons nucléaires ou qui sont reliés entre eux par un chaînon de pontage, au moins 2 segments nucléaires de formule:

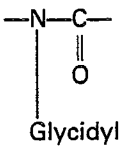

ou au moins un segment nucléaire de formule:

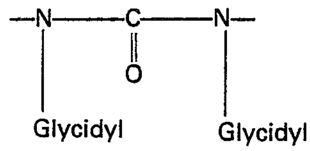

dans lesquels le radical glycidyle répond à la formule

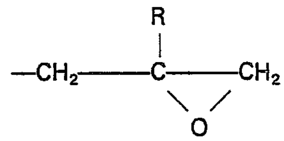

dans laquelle R est de l'hydrogène ou le radical méthyle, et en ce qu'en outre, pour autant que ces segments nucléaires ne soient pas eux-mêmes déja fermés, ils contiennent du carbone et éventuellement en plus de l'azote comme chaînons nucléaires, et en ce qu'ils peuvent présenter comme autres substituants sur les noyaux hétérocycliques des halogènes ou des radicaux hydrocarbonés non substitués n'ayant pas plus de 8 atomes de carbone, en exceptant en tant qu'hétérocycles les isocyanurates, les urazols et les hydantoïnes, tandis que les N-hétérocycles polyglycidyl-substitués qui y sont présents ne dépassent pas le poids moléculaire de 1000.

2. Procédé de fabrication de préparations médicamenteuses selon la revendication 1, caractérisé en ce que les matières actives cytostatiques sont choisies à partir de composés hétérocycliques présentant deux à trois groupes glycidyle appartenant au groupe des uréides, comme des uraciles, glycolurites ou benzimidazolones, des polycarboximides et des dérivés d'acide barbiturique.

3. Procédé de fabrication de préparations médicamenteuses selon revendications 1 à 2, caractérisé en ce que les matières actives cytostatiques présentent seulement un radical N-glycidyle dans la forme imide ou amide dans un noyau et au moins un autre radical N-glycidyle dans un autre noyau hétérocyclique de la même molécule, ces noyaux étant reliés ou fusionnés entre eux.

4. Procédé de fabrication de préparations médicamenteuses selon l'une des revendications 1 à 3, caractérisé en ce que les radicaux N-glycidyle font partie de noyaux hétérocycliques ayant jusqu'à 10 chaînons nucléaires, de préférence 4 à 7 chaînons nucléaires et en particulier 5 et/ou 6 chaînons nucléaires.

5. Procédé de fabrication de préparations médicamenteuses selon l'un des revendications 1 à 4, caractérisé en ce qu'elles contiennent des N-hétérocycles substitués par plusieurs radicaux glycidyle qui présentent comme substituants sur le (les) noyau (x) hétérocyclique (s) des radicaux hydrocarbonés qui possèdent chacun jusqu'à 8 atomes de carbone.

6. Procédé de fabrication de préparations médicamenteuses selon l'une des revendications 1 à 5, contenant des composés formule générale II:

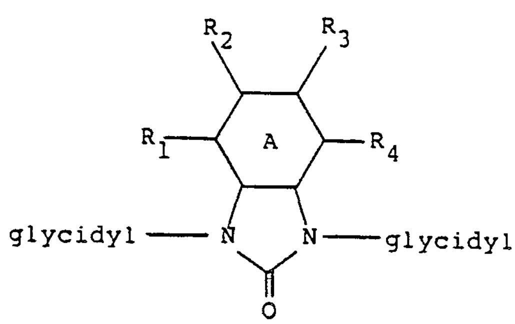

(II)

dans laquelle glycidyl a la signification indiquée, A est un système avec noyau en 6, aromatique, cycloaliphatique ou oléfiniquement insaturé, qui peut aussi contenir un hétéroatome, les radicaux $R_1$ à $R_4$ ayant la signification suivante: des radicaux in identiques ou différents appartenant au groupe hydrogène, halogène, radicaux hydrocarbonés ayant chacun au plus 8 atomes de carbone.

7. Procédé de fabrication de préparations médicamenteuses selon l'une des revendications 1 à 5, contenant des composés de formule générale III:

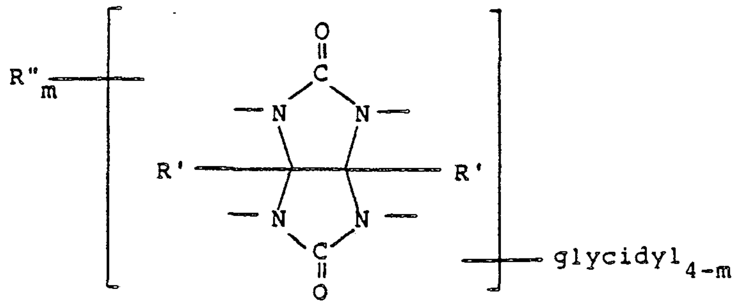

(III)

dans laquelle glycidyl a la signification indiquée et les radicaux R', R'' et m signifient ce qui suit:

R': radicaux identiques ou différents du groupe hydrogène, radicaux hydrocarbonés ayant chacun jusqu'à 8 atomes de carbone

R'': radicaux identiques ou différents du groupe hydrogène, radicaux hydrocarbonés ayant chacun jusqu'à 4 atomes de carbone

m: 0, 1 ou 2.

8. Procédé de fabrication de préparations médicamenteuses selon l'une des revendications 1 à 5, contenant des composés de formule générale IV

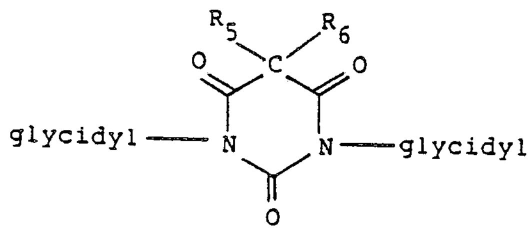

(IV)

dans laquelle les radicaux $R_5$ et $R_6$ ont la signification suivante:

$R_5$ et $R_6$:

(1) des radicaux identiques ou différents du groupe hydrogène et radicaux hydrocarbonés ayant chacun jusqu'à 12 atomes de carbone

(2) $R_5$ et $R_6$ sont cyclisés en commun en un système nucléaire.

9. Procédé de fabrication de préparations médicamenteuses selon l'une des revendications 1 à 5, contenant des composés de formule générale V

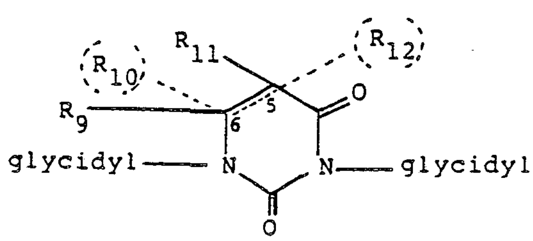

(V)

dans laquelle des radicaux $R_9$, $R_{10}$, $R_{11}$ et $R_{12}$ ont la signification suivante:

$R_9$ à $R_{12}$:

(1) des radicaux identiques ou différents du groupe hydrogène, halogène et radicaux hydrocarbonés ayant chacun jusqu'à 8 atomes de carbone, qui peuvent aussi être substitués

(2) au moins deux des radicaux $R_9$ à $R_{12}$ sont cyclisés en commun en un système nucléaire

(3) au moins une des paires $R_9$/$R_{10}$ ou $R_{11}$/$R_{12}$ forment ensemble le radical

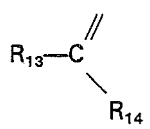

dans lequel $R_{13}$ et $R_{14}$ ont la signification de $R_9$ à $R_{12}$ indiquée en (1) ou en (2), tandis que glycidyl a la signification citée.

10. Procédé de fabrication de préparations médicamenteuses selon l'une des revendications 1 à 5, caractérisé en ce qu'elles contiennent des polycarboximides avec au moins 2 groupes imide cycliques substitués par des radicaux glycidyle, choisies dans le groupe:

diimide d'acide N,N'-diglycidyl-pyromellitique (diimide d'acide 1,2,4,5-benzène-tétracarboxylique)

diimide d'acide N,N'-diglycidyl-1,2,3,4-benzène-tétracarboxylique)
diimide d'acide N,N'-diglycidyl-1,4,5,8-naphtalène tetracarboxylique
diimide d'acide N,N'-diglycidyl-2,3,4,5-pyridine-tetracarboxylique
diimide d'acide N,N'-diglycidyl-2,3,5,6-pyridine-tetracarboxylique
diimide d'acide N,N'-diglycidyl-1,2,4,5-cyclohexane-tetracarboxylique
diimide d'acide N,N'-diglycidyl-1,1,2,2-éthane-tetracarboxylique
triimide d'acide N,N'-diglycidyl-1,2,3,4-butane-tetracarboxylique
triimide d'acide N,N',N''-triglycidyl-benzène-hexacarboxylique
diimide d'acide N,N'-diglycidyl-tétrahydrofuranne-tetracarboxylique.

11. Procédé de fabrication de préparations médicamenteuses selon l'une des revendications 1 à 10, caractérisé en ce qu'en cas de présence de radicaux hydrocarbonés comme substituants sur le ou les noyaux hétérocycliques, ces radicaux hydrocarbonés dans leur ensemble ne présentent pas plus de 12 atomes de carbone, en particulier pas plus de 8 atomes de carbone, pouvant être particulièrement préférés les radicaux ayant jusqu'à 4 atomes de carbone.

12. Procédé de fabrication de préparations médicamenteuses selon l'une des revendications 1 à 11, caractérisé en ce les substituants présents sur le système nucléaire hétérocyclique en tout ne présentent pas plus de 12 atomes de carbone, en particulier pas plus de 8 atomes de carbone, l'occurrence pouvant être particulièrement préféré le fait que ces radicaux en tout ne possèdent pas plus de 4 atomes de carbone.

13. Procédé de fabrication de préparations médicamenteuses selon l'une des revendications 1 à 12, caractérisé en ce qu'elles contiennent des composés polyglycidyliques ayant 2 à 4, en particulier 2 ou 3 radicaux glycidyle sur les atomes N dans la liaison amide et/ou imide.

14. Procédé de fabrication de préparations médicamenteuses selon l'une des revendications 1 à 13, caractérisé en ce qu'elles contiennent les N-hétérocycles polyglycidyl-substitués en mélange avec les substances auxiliaires et/ou de support en usage pharmacologique.